# EUROPÄISCHE PATENTSCHRIFT

(11) **EP 0 457 726 B1**
(45) Veröffentlichungstag und Bekanntmachung des Hinweises auf die Patenterteilung: **07.02.1996**
(21) Anmeldenummer: 91810354.0
(22) Anmeldetag: 08.05.1991
(51) Int. Cl.: C07D 239/42, A01N 43/54

(54) **2-Anilino-pyrimidin Derivate als Schädlingsbekämpfungsmittel**
2-Anilinopyrimidine derivatives as pesticides
Dérivés d'anilino-2-pyrimidine comme pesticides

(30) Priorität: 17.05.1990 CH 1668/90
(43) Veröffentlichungstag der Anmeldung: 21.11.1991
(73) Patentinhaber: CIBA-GEIGY AG, CH-4002 Basel (CH)
(72) Erfinder: Hubele, Adolf, Dr., CH-4312 Magden (CH)

(56) Entgegenhaltungen:
- EP-A- 0 135 472
- EP-A- 0 310 550
- EP-A- 0 337 943

## Beschreibung

Die vorliegende Erfindung betrifft neue 2-Anilino-pyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem von schädlichen Insekten und pflanzenschädigenden Mikroorganismen, vorzugsweise Pilzen.

Die erfindungsgemässen Verbindungen entsprechen der allgemeinen Formel I 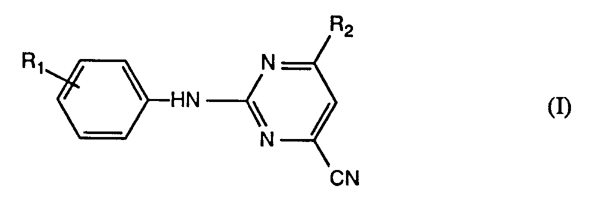
in welcher bedeuten:
R₁ Wasserstoff, 3-Fluor oder 4-Fluor; und
R₂ C₁-C₄-Alkyl, durch Halogen oder Hydroxy substituiertes C₁-C₂-Alkyl, C₃-C₆-Cycloalkyl oder durch Methyl und/oder Halogen ein- bis dreifach gleich oder verschieden substituiertes C₃-C₆-Cycloalkyl; unter Einschluss ihrer Säureadditionssalze und Metallsalzkomplexe.

Unter Alkyl sind je nach Anzahl der benannten Kohlenstoffatome beispielsweise zu verstehen Methyl, Ethyl, Propyl, Butyl, sowie ihre Isomeren, wie z.B. Isopropyl, Isobutyl, tert.-Butyl oder sek.-Butyl. Halogen steht für Fluor, Chlor, Brom oder Jod, vorzugsweise für Chlor. Cycloalkyl bedeutet je nach Zahl der genannten Kohlenstoffatome z.B. Cyclopropyl, Cyclobutyl, Cyclopentyl oder Cyclohexyl.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicyclsäure, 2-Phenoxybenzoesäure, 2-Acetoxybenzoesäure oder 1,2-Naphthalin-disulfonsäure.

Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicyclaten, Benzoaten usw. der Elemente der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Eisen, Kobalt, Nickel, Kupfer, Zink usw. Bevorzugt sind die Nebengruppen-Elemente der 4. Periode. Die Metalle können dabei in den verschiedenen ihnen zukommenden Wertigkeiten vorliegen. Die Metallkomplexe können ein- oder mehrkernig auftreten, d.h. sie können ein oder mehrere organische Molekülanteile als Liganden enthalten.

Die Verbindungen der Formel I sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende fungizide Wirkung, sondern auch durch besonders gute Pflanzenverträglichkeit aus.

Folgende Wirkstoffgruppen sind aufgrund ihrer ausgeprägten mikrobiziden, insbesondere pflanzenfungiziden, Aktivität bevorzugt.

Gruppe 1: Verbindungen der Formel I, worin bedeuten:
R₁ Wasserstoff, 3-Fluor oder 4-Fluor, und
R₂ C₁-C₃-Alkyl, Cyclopropyl oder mit Methyl oder Chlor substituiertes Cyclopropyl.

Gruppe 2: Verbindungen der Formel I, worin bedeuten:
R₁ Wasserstoff; und
R₂ Methyl, Ethyl, Cyclopropyl oder 2-Methylcyclopropyl.

Gruppe 3: Verbindungen der Formel I, worin bedeuten:
R₁ Wasserstoff, 3-Fluor oder 4-Fluor; und
R₂ Methyl, Ethyl oder Cyclopropyl.

Für den Einsatz als fungizide Wirkstoffe sind als besonders bevorzugt folgende Verbindungen zu nennen:
2-Phenylamino-4-cyano-6-methyl-pyrimidin (Verb. Nr. 1.1),
2-Phenylamino-4-cyano-6-ethyl-pyrimidin (Verb. Nr. 1.3),
2-Phenylamino-4-cyano-6-cyclopropyl-pyrimidin (Verb. Nr. 1.11) und
2-(3-Fluorphenylamino)-4-cyano-6-methyl-pyrimidin (Verb. Nr. 1.16).

N-Pyrimidinylanilinverbindungen sind bereits bekannt. So sind in der publizierten europäischen Patentanmeldung EP-A-0 224 339, EP-A-0 310 550 und der DD-Patentschrift 151 404 Verbindungen, die eine N-2-Pyrimidinylstruktur aufweisen, als wirksarm gegen pflanzenschädigende Fungi beschrieben. Daneben sind Verbindungen bekannt geworden (vgl. europäische Patentanmeldung EP-A-0 337 943 und EP-A-0 135 472), die über eine mit Cyanogruppen substituierte Anilinopyrimmidin-Struktur verfügen. Diese dienen gemäss Beschreibung jedoch lediglich als Zwischenprodukte für die Synthese von herbizid-wirksamen Harnstoffderivaten. Die bekannten N-Pyrimidinylanilin-Derivate haben bisher die an sie gestellten Forderungen für den Einsatz als Fungizide, vor allem in geringer Aufwandmenge nicht befriedigen können.

Die Verbindungen der Formel I werden hergestellt, indem man
1.1 ein Guanidinsalz der Formel IIa 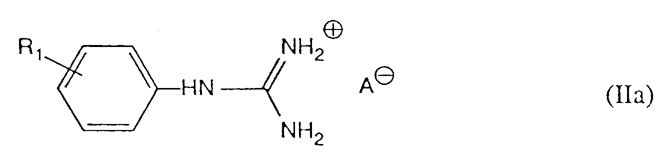 oder eine Guanidinverbindung der Formel IIb 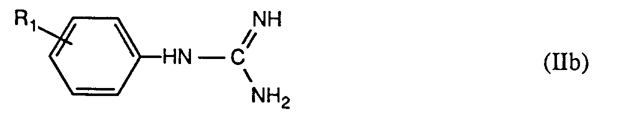 mit einem Keton der Formel III 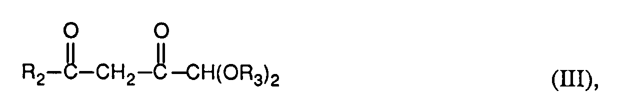 worin R₃ ein Alkyl, bevorzugt C₁-C₄-Alkyl, bedeutet und A^{⊖} ein Säureanion darstellt, in einem protischen Lösungsmittel oder ohne Lösungsmittel bei Temperaturen von 40° bis 160°C, bevorzugt 60° bis 110°C, zu einer Pyrimidinverbindung der Formel IV 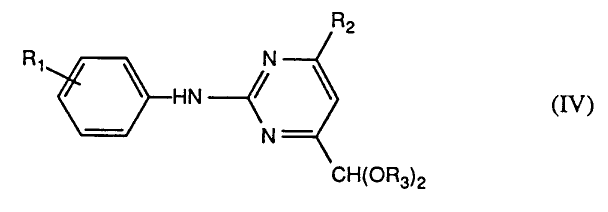 umsetzt und
1.2 das erhaltene Acetal der Formel IV in Gegenwart einer Säure, z.B. einer Halogenwasserstoffsäure oder Schwefelsäure, in Wasser oder wässrigen Gemischen von inerten Lösungsmitteln, z.B. Alkoholen oder Dimethylformamid, bei Temperaturen von 20° bis 100°C, bevorzugt 30° bis 60°C, zum Pyrimidinylaldehyd der Formel V 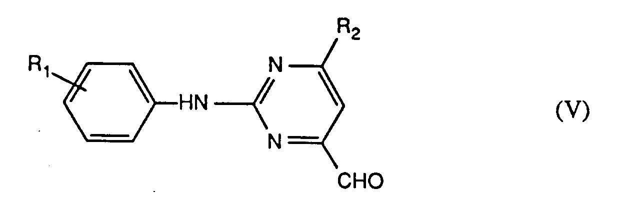 hydrolysiert und
1.3 anschliessend den Aldehyd der Formel V mit einem Hydroxylaminsalz und einer Base, die das Hydroxylamin freisetzt, in einem protischen Lösungsmittel, bevorzugt in Gegenwart von Wasser, bei Temperaturen von 10° bis 90°C, bevorzugt bei 10°C bis 60°C, in das Oxim der Formel VI 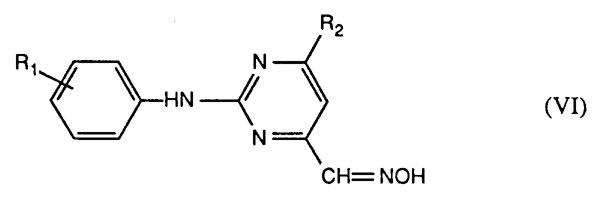 überführt und
1.3.1 das Oxim der Formel VI einer Wasserabspaltung unterwirft; wobei das Oxim z.B. intermediär zu einer Verbindung der Formel VII 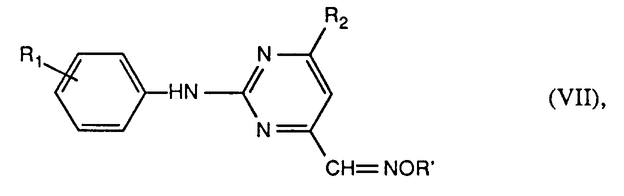 worin R' einen Acylrest COR'', einen Carbamoylrest CONHR'' oder einen Oxycarbonylrest COOR'' darstellt, in welchem R'' einen Alkylrest, bevorzugt C₁-C₄-Alkyl bedeutet, umgewandelt wird und anschliessend die Verbindung der Formel VII durch Abspaltung des Acylrestes HOCOR'' in inerten Lösungsmitteln bei Temperaturen von 60° bis 120°C, bevorzugt 60° bis 100°C, zersetzt; oder
1.4 aus dem Pyrimidinaldehyd der Formel V durch Umsetzung in tertiären Basen, z.B. Pyridin und Hydroxylaminsalz, intermediär das Oxim der Formel VI herstellt und daraus durch Behandlung mit einem Säurehalogenid oder Säureanhydrid, z.B. Essigsäureanhydrid, bei Temperaturen von 40° bis 120°C, bevorzugt 60° bis 100°C, in situ direkt die Verbindung der Formel I erhält; oder
2. eine Verbindung der Formel VIII 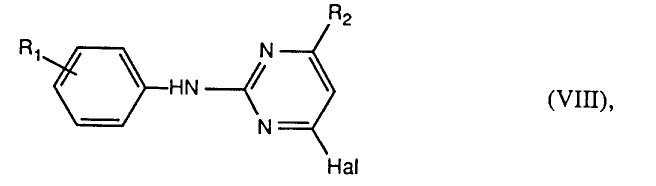 mit einem Cyanid der Formel IX

   Me-CN (IX),

   worin Hal Fluor, Chlor, Brom oder Jod, bevorzugt Chlor oder Jod, bedeutet, und Me ein Alkali-, Erdalkali- oder Schwermetallkation, vorzugsweise Natrium, Kalium oder Kupfer, darstellt, in einem aprotischen Lösungsmittel, z.B. Dimethylformamid oder Dimethylsulfoxid, bei Temperaturen von 60° bis 160°C, bevorzugt 80° bis 120°C, umsetzt; oder
3.1 Harnstoff der Formel X 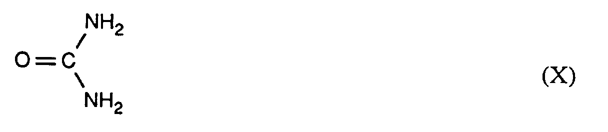 mit einem Diketon der Formel III 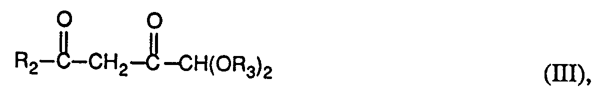 worin R₃ ein Alkyl, bevorzugt C₁-C₄-Alkyl, bedeutet, in Gegenwart einer Säure in einem inerten Lösungsmittel bei Temperaturen von 20° bis 140°C, bevorzugt 20° bis 40°C, zu einer Pyrimidinverbindung der Formel XI 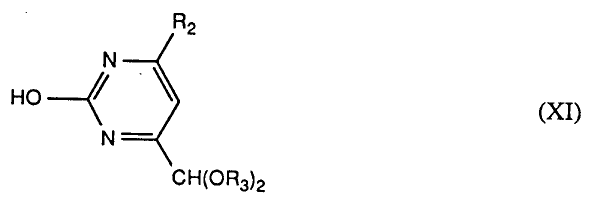 cyclisiert und
3.2 die OH-Gruppe der Verbindung der Formel XI mit überschüssigem PO(Hal)₃ in Gegenwart oder Abwesenheit eines inerten Lösungsmittels bei Temperaturen von 50° bis 110°C, bevorzugt bei der Rückflusstemperatur des PO(Hal)₃, gegen Halogen austauscht und eine Verbindung der Formel XII 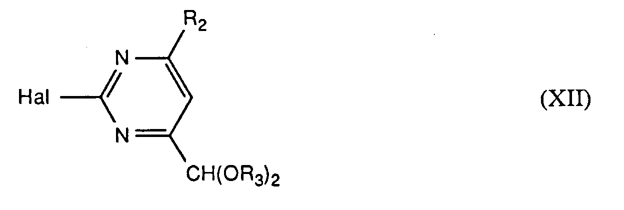 erhält, wobei Hal Halogen, bevorzugt Chlor oder Brom, bedeutet und weiter
3.3 eine Verbindung der Formel XII mit einer Anilinverbindung der Formel XIII 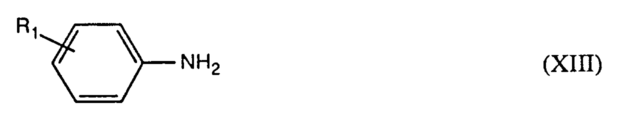 entweder
   a) in Gegenwart eines Protonenakzeptors, z.B. im Ueberschuss der Anilinverbindung der Formel XIII oder einer anorganischen Base, mit oder ohne inertes Lösungsmittel oder
   b) in Gegenwart einer Säure in einem inerten Lösungsmittels bei jeweils Temperaturen von 60° bis 120°C, bevortugt 80° bis 100°C, zu einer Verbindung der Formel IV 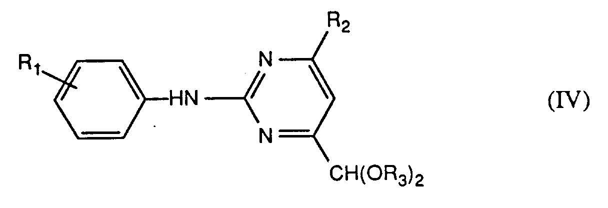 umsetzt und das erhaltene Acetal wie unter 1.2, 1.3 und 1.3.1 beschrieben in eine Verbindung der Formel I überführt, wobei in den vorgängig beschriebenen Verfahren die Reste R₁ und R₂ die unter Formel I angegebenen Bedeutungen besitzen.

In den beschriebenen Verfahren kommen bei den Verbindungen der Formel IIa für das Säureanion A^{⊖} beispielsweise folgende Salzreste in Betracht: Carbonat, Hydrogencarbonat, Nitrat, Halogenid, Sulfat oder Hydrogensulfat.

Unter Halogenid sind jeweils Fluorid, Chlorid, Bromid oder Jodid, bevorzugt Bromid oder Chlorid, zu verstehen.

Als Säuren finden vornehmlich anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsäure oder Salpetersäure Verwendung; jedoch können auch geeignete organische Säuren wie Essigsäure, Toluolsulfonsäure u.a. verwendet werden.

Als Protonenakzeptoren dienen z.B. anorganische oder organische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride wie z.B. Natriumhydrid. Als organische Basen seien beispielsweise tertiäre Amine wie Triethylamin, Triethylendiamin, Pyridin genannt.

In den vorstehend beschriebenen Verfahren können in Anpassung an die jeweiligen Reaktionsbedingungen beispielsweise folgende Lösungsmittel neben den bereits genannten verwendet werden.

Halogenkohlenwasserstoffe, z.B. Chlorkohlenwasserstoffe, wie Tetrachlorethan, Methylenchlorid, Chloroform, Tetrachlorkohlenstoff, Chlorbenzol, Chlortoluol, Ether, wie Ethylpropylether, Methyl-tert.-butylether, Di-n-butylether, Di-isobutylether, Diisoamylether, Diisopropylether, Diethylether, Ethylenglykoldimethylether, Tetrahydrofuran, Dioxan, Nitrokohlenwasserstoffe wie Nitrobenzol, Nitrile wie Acetonitril, Butyronitril, aliphatische oder cycloaliphatische Kohlenwasserstoffe, wie Heptan, Hexan, Benzinfraktionen innerhalb eines Siedepunktintervalles von 70°C bis 190°C, Cyclohexan, Petrolether, Ligroin, Ester wie Ethylacetat, Amide, z.B. Formamid, Dimethylformamid; Ketone, wie Aceton, Methylethylketon; Alkohole, insbesondere niedere aliphatische Alkohole, wie z.B. Methanol, Ethanol, n-Propanol, iso-Propanol sowie die Isomeren der Butanole; Wasser. Auch Gemische der genannten Lösungs- und Verdünnungsmittel kommen in Betracht.

Analoge Synthesemethoden zu den vorstehend beschriebenen Herstellungsverfahren sind in der Literatur publiziert. Als Hinweise seien genannt: A.Kreutzberger und J.Gillessen, J.Heterocyclic Chem.22, 101 (1985). O. Stark, Ber.Dtsch.Chem.Ges 42, 699 (1909); J. Hale, J.Am.Chem.Soc.36, 104 (1914); G.M. Kosolapoff, J.Org.Chem.26, 1895 (1961); St. Angerstein, Ber.Dtsch.Chem.Ges.34, 3956 (1901); G.M. Kosolapoff, J.Org.Chem.26, 1895 (1961); M.P.V. Boarland and J.F.W. McOmie, J.Chem.Soc. 1951, 1218; T.Matsukawa und K. Shirakuwa, J.Pharm.Soc.Japan 71, 933 (1951); Chem.Abstr.46, 4549 (1952).

Die beschriebenen Herstellungsverfahren einschliesslich aller Teilschritte sind ein Bestandteil der vorliegenden Erfindung.

Weiterhin sind die neuen Zwischenprodukte der Formel V 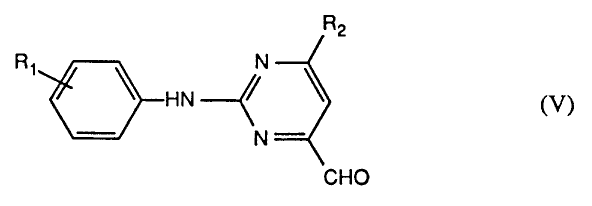
worin R₁ Wasserstoff, 3-Fluor oder 4-Fluor und R₂ C₁-C₄-Alkyl oder durch Halogen oder Hydroxy substituiertes C₁-C₂-Alkyl bedeuten, sowie die Zwischenprodukte der Formel VI 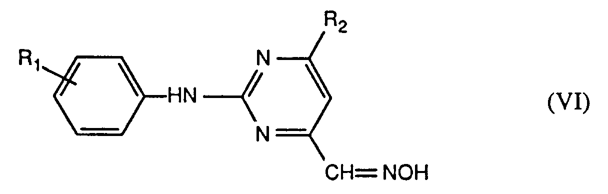
worin R₁ Wasserstoff, 3-Fluor oder 4-Fluor und R₂ C₁-C₄-Alkyl, durch Halogen oder Hydroxy substituiertes C₁-C₂-Alkyl, C₃-C₆-Cycloalkyl oder durch Methyl und/oder Halogen ein- bis dreifach gleich oder verschieden substituiertes C₃-C₆-Cycloalkyl bedeuten, ein Bestandteil der vorliegenden Erfindung.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen für die direkte Pflanzenbehandlung oder die Bodenbehandlung liegen im allgemeinen bei 50 g bis 5 kg Aktivsubstanz (AS) je ha, bevorzugt 100 g bis 2 kg AS/ha, insbesondere bei 200 g bis 600 g AS/ha. Die Mengen an Wirkstoff für gebeiztes und danach ausgesätes Saatgut liegen deutlich niedriger bei umgerechnet 0,1 g AS/ha bis 500 g AS/ha, d.h. diejenigen Mengen an Wirkstoff, die sich zusammen mit dem Saatgut auf 1 ha Ackerfläche befinden. Bevorzugt sind hierbei 0,5 g AS/ha bis 100 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung

### 1. Herstellungsbeispiele

### Beispiel 1.1 Herstellung des Zwischenprodukts 2-Anilino-4-formyldiethylacetal-6-methyl-pyrimidin

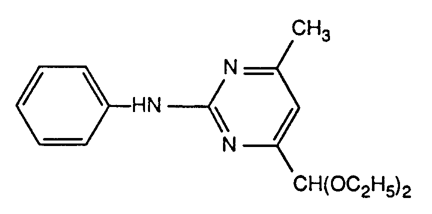

11,8 g (60 mMol) Phenylguanidin-hydrogencarbonat und 12 g (64 mMol) 1-Methyl-3-formyldiethylacetal-1,3-propandion in 80 ml Ethanol werden unter Rühren 4 Stunden unter Rückfluss erhitzt, wobei die Kohlendioxidentwicklung mit fortschreitender Reaktionsdauer nachlässt. Nach dem Abkühlen wird das Ethanol weitgehend verdampft, der Rückstand in 30 ml Diethylether aufgenommen, dreimal mit je 30 ml Wasser gewaschen, über Natriumsulfat getrocknet, filtriert und der Diethylether verdampft. Das zurückbleibende dunkelbraune Oel wird in 350 ml Petrolether (Sdp. 40-60°C) in der Wärme gelöst, in Gegenwart von Aktivkohle 10 Minuten unter Rückfluss erhitzt, nach dem Abkühlen auf ca. 40°C über Hyflo filtriert und auf ein Volumen von etwa 30 ml eingeengt. Die ausgefallenen gelblichen Kristalle werden abfiltriert und schmelzen bei 63-65°C.

### Beispiel 1.2 Herstellung des Zwischenprodukts 2-Anilino-4-formyl-6-methyl-pyrimidin

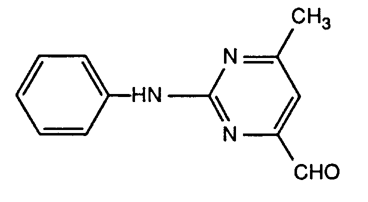

3 g (10,4 mMol) 2-Anilino-4-formyldiethylacetal-6-methyl-pyrimidin und 1 g konz. Salzsäure in 30 ml Wasser werden 24 Stunden bei 45°C gerührt, auf 15°C gekühlt und mit Natriumbicarbonatlösung der pH-Wert auf 8 eingestellt. Das hellbraune Pulver wird abfiltriert, mit Wasser nachgewaschen und aus 70 ml Acetonitril umkristallisiert. Die gelben Kristalle schmelzen bei 130-131°C.

### Beispiel 1.3 Herstellung von 2-Phenylamino-4-cyano-6-methyl-pyrimidin

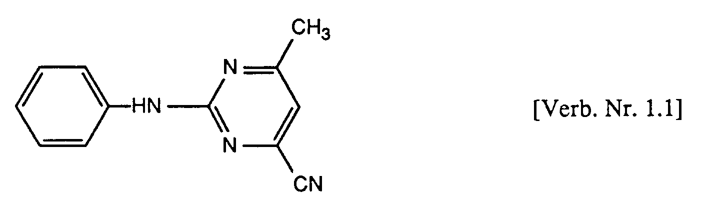

21,3 g (0,1 Mol) 2-Anilino-4-formyl-6-methyl-pyrimidin in 90 ml Pyridin werden unter Rühren portionenweise innerhalb von 5 Minuten mit 11,8 g (0,17 Mol) Hydroxylaminhydrochlorid versetzt, wobei die Temperatur auf 40°C ansteigt. Nach einhalbstündigem Rühren bei 50°C werden innerhalb einer viertel Stunde 40 ml Acetanhydrid zugetropft, wobei die Temperatur bis auf 70°C ansteigt. Nach dreistündigem Rühren bei 90°C wird nach dem Abkühlen das Reaktionsgemisch im Vakuum eingeengt und der dunkelbraune, ölige Rückstand auf 600 ml Eiswasser gegossen und mit verdünnter Natronlauge auf pH 8 gebracht. Nach dreimaliger Extraktion mit je 150 ml Ethylacetat werden die vereinigten Extrakte zweimal mit je 50 ml Wasser gewaschen, über Natriumsulfat getrocknet, filtriert und das Lösungsmittel verdampft. Der dunkelbraune, ölige Rückstand wird in 200 ml Tetrahydrofuran gelöst, mit Aktivkohle behandelt, filtriert und das Lösungsmittel verdampft. Der zurückbleibende, bräunlich gefärbte Feststoff wird zweimal aus Diisopropylether/Methanol umkristallisiert. Das gelbe Kristallpulver schmilzt zwischen 125-126°C.

### Beispiel 1.4 Herstellung des Zwischenprodukts 2-Anilino-4-oximino-6-cyclopropyl-pyrimidin

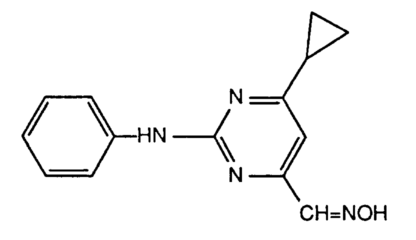

10 g (42 mMol) 2-Anilino-4-formyl-6-cyclopropylpyrimidin in 150 ml Ethanol werden unter Rühren bei Raumtemperatur innerhalb einer Viertelstunde portionenweise zunächst mit 3,6 g (52 mMol) Hydroxylaminhydrochlorid, dann mit 2,8 g (26,4 mMol) Soda in 15 ml Wasser versetzt und anschliessend 24 Stunden bei Raumtemperatur gerührt. Die gelblich gefärbte Suspension wird mit 50 ml Wasser verdünnt, filtriert und mit 10 ml Wasser nachgewaschen und der gelbe semikristalline Rückstand aus 120 ml Ethanol umkristallisiert. Die beigen Kristalle schmelzen bei 175-185°C.

### Beispiel 1.5 Herstellung von 2-Phenylamino-4-cyano-6-cyclopropyl-pyrimidin

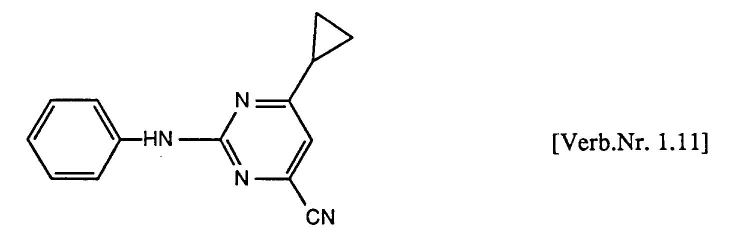

Zu 7,6 g (30 mMol) 2-Anilino-4-oximino-6-cyclopropyl-pyrimidin und 200 mg 1,4-Diazabicyclo[2.2.2]octan in 60 ml Dioxan werden innerhalb von 10 Minuten bei Raumtemperatur unter Rühren 3 g (53 mMol) Methylisocyanat in 10 ml Dioxan zugetropft. Nach 16-stündigem Rühren bei 75°C wird abgekühlt, das Lösungsmittel verdampft und das zurückbleibende hellbraune Oel säulenchromatographisch über Kieselgel (Toluol/Diethylether: 3/2) gereinigt. Nach dem Abdampfen des Laufmittelgemisches wird der gelbe Festkörper durch Umkristallisation aus Diisopropylether gereinigt. Die gelben Kristalle schmelzen bei 122-125°C.

Auf diese Art oder nach einer der weiter oben angegebenen Methoden lassen sich folgende Verbindungen der Formel I herstellen.

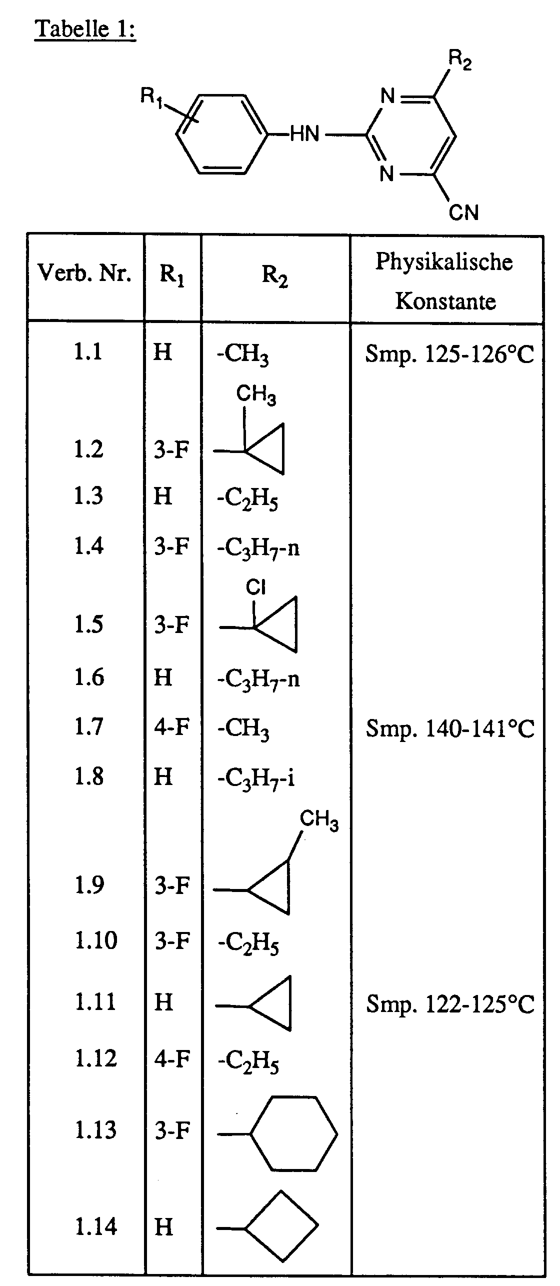

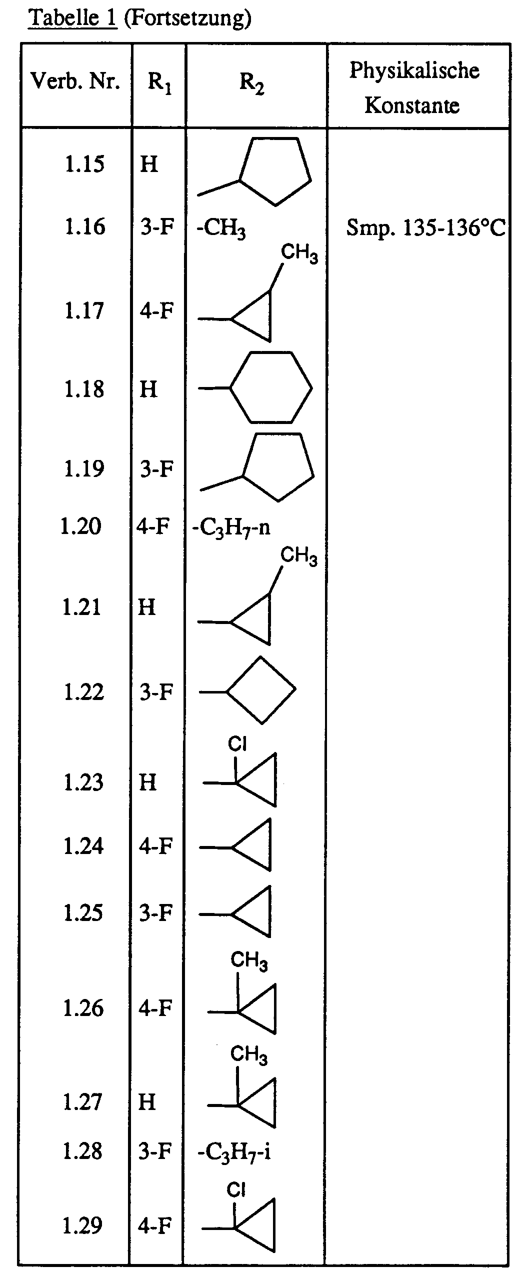

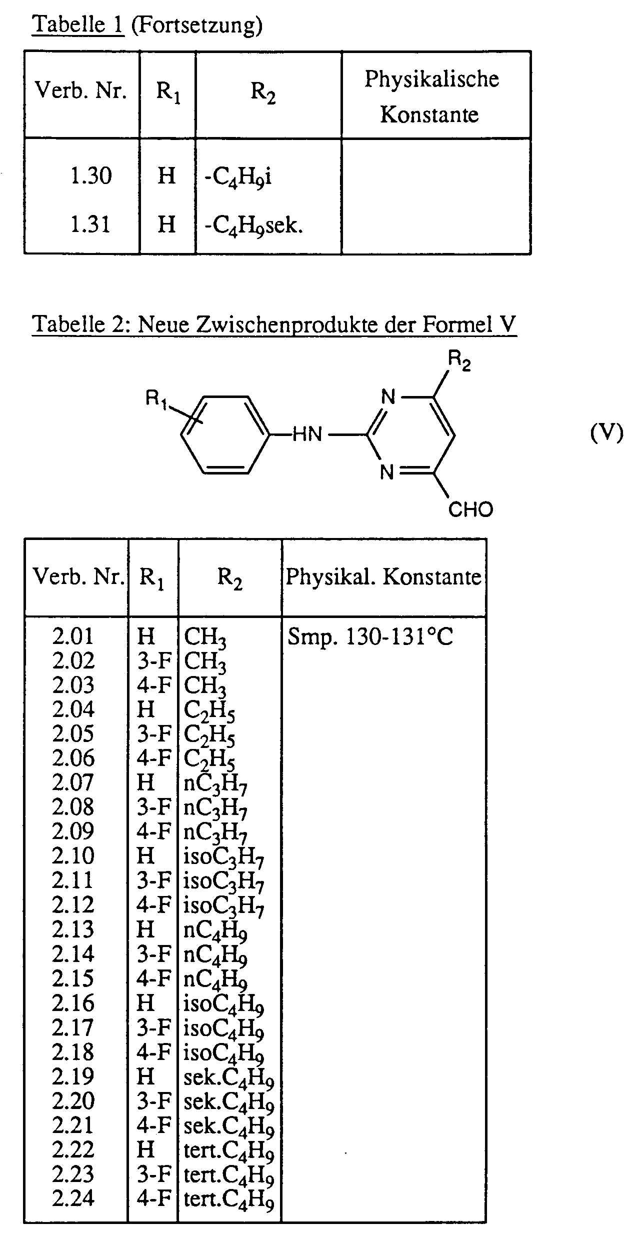

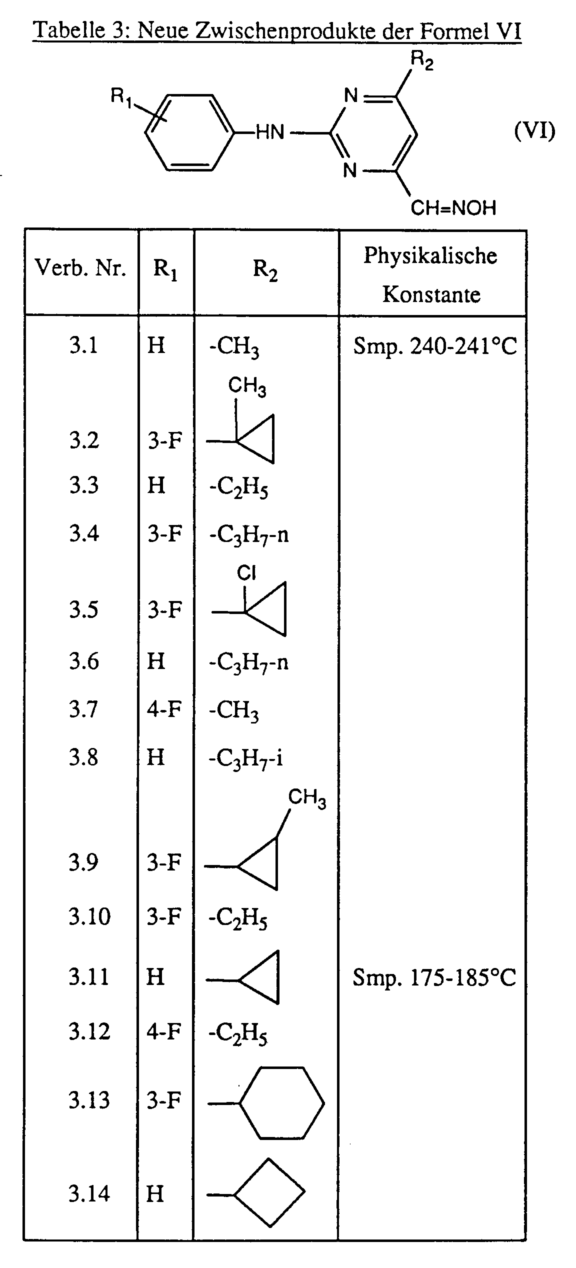

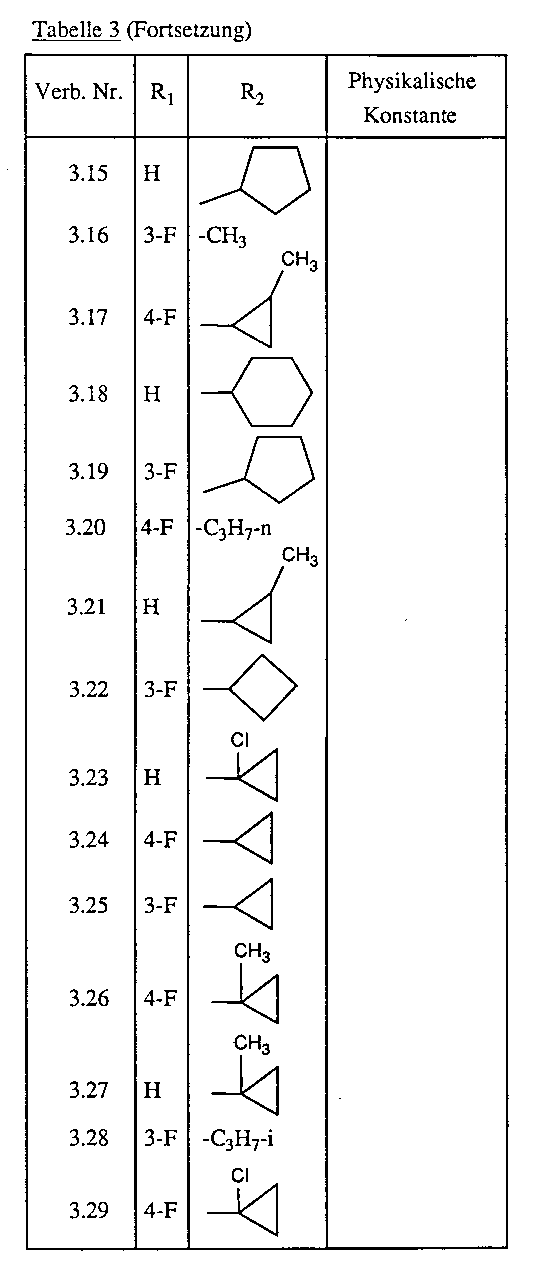

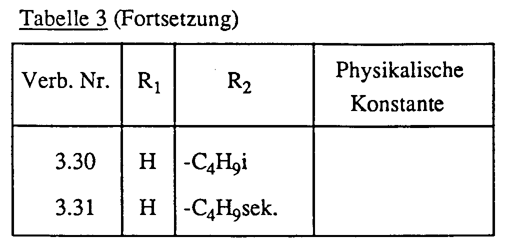

### 2. Formulierungsbeispiele für feste Wirkstoffe der Formel I (% = Gewichtsprozent)

| 2.1. Spritzpulver | a) | b) | c) |
|---|---|---|---|
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wilkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2. Emulsions-Konzentrat | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.4. Extruder Granulat | |
|---|---|
| Wirkstoff aus der Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5. Umhüllungs-Granulat | |
|---|---|
| Wirkstoff aus der Tabelle 1 | 3 % |
| Polyethylenglykol (MG 200) | 3 % |
| Kaolin (MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6. Suspensions-Konzentrat | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Venturia inaequalis auf Apfeltrieben

### Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Verbindungen aus Tabelle 1 zeigen gegen Venturia gute Wirksamkeit (Befall: weniger als 20 %). Unbehandelte aber infizierte Kontrollpflanzen weisen dagegen einen Venturia-Befall von 100 % auf.

### Beispiel 3.2: Wirkung gegen Botrytis cinerea an Apfelfrüchten

### Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus Tabelle 1 zeigen gegen Botrytis gute Wirksamkeit (Befall: weniger als 20 %). So reduzierten z.B. die Verbindungen Nr. 1.1 und 1.11 den Botrytis-Befall auf 0 bis 10 %. Unbehandelte aber infizierte Kontrollpflanzen weisen dagegen einen Botrytis-Befall von 100 % auf.

### Beispiel 3.3: Wirkung gegen Erysiphae graminis auf Gerste

### a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus Tabelle 1 zeigen gegen Erysiphae gute Wirksamkeit (Befall: weniger als 20 %). Unbehandelte aber infizierte Kontrollpflanzen weisen dagegen einen Erysiphae-Befall von 100 % auf.

### Beispiel 3.4: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 % Pilzbefall).

### Beispiel 3.5 Wirkung gegen Colletotrichum lagenarium auf Gurken

Gurkenpflanzen werden nach 2-wöchiger Anzucht mit einer aus Spritzpulver des Wirkstoffs hergestellten Spritzbrühe (Konzentration 0,002 %) besprüht. Nach 2 Tagen werden die Pflanzen mit einer Sporensuspension (1,5x10⁵ Sporen/ml) des Pilzes infiziert und für 36 Stunden bei 23°C und hoher Luftfeuchtigkeit inkubiert. Die Inkubation wird dann bei normaler Luftfeuchtigkeit und ca. 22-23°C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen aus Tabelle 1 zeigen gute Wirksamkeit und verhindern die Ausbreitung des Krankheitsbefalls. Der Pilzbefall wird auf 20 % oder weniger zurückgedrängt.

## Patentansprüche (Patentansprüche für folgende(n) Vertragsstaat(en): AT, BE, CH, DE, DK, FR, GB, GR, IT, LI, LU, NL, SE)

1. Verbindungen der Formel I 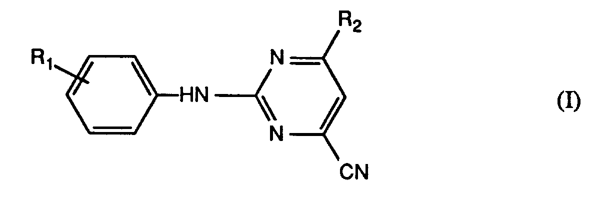 in welcher bedeuten:
R₁ Wasserstoff, 3-Fluor oder 4-Fluor; und
R₂ C₁-C₄-Alkyl, durch Halogen oder Hydroxy substituiertes C₁-C₂-Alkyl, C₃-C₆-Cycloalkyl oder durch Methyl und/oder Halogen ein- bis dreifach gleich oder verschieden substituiertes C₃-C₆-Cycloalkyl; unter Einschluss ihrer Säureadditionssalze und Metallsalzkomplexe.

2. Verbindungen der Formel I gemäss Anspruch 1, in welcher bedeuten:
R₁ Wasserstoff, 3-Fluor oder 4-Fluor; und
R₂ C₁-C₃-Alkyl, Cyclopropyl oder mit Methyl oder Chlor substituiertes Cyclopropyl.

3. Verbindungen der Formel I gemäss Anspruch 1, in welcher bedeuten:
R₁ Wasserstoff; und
R₂ Methyl, Ethyl, Cyclopropyl oder 2-Methylcyclopropyl.

4. Verbindungen der Formel I gemäss Anspruch 1, in welcher bedeuten:
R₁ Wasserstoff, 3-Fluor oder 4-Fluor; und
R₂ Methyl, Ethyl oder Cyclopropyl.

5. Eine Verbindung der Formel I gemäss Anspruch 4 ausgewählt aus der Gruppe:
2-Phenylamino-4-cyano-6-methyl-pyrimidin (Verb. Nr. 1.1),
2-Phenylamino-4-cyano-6-ethyl-pyrimidin (Verb. Nr. 1.3),
2-Phenylamino-4-cyano-6-cyclopropyl-pyrimidin (Verb. Nr. 1.11) und
2-(3-Fluorphenylamino)-4-cyano-6-methyl-pyrimidin (Verb. Nr. 1.16).

6. Verfahren zur Herstellung der Verbindungen der Formel I, dadurch gekennzeichnet, dass man
1.1 ein Guanidinsalz der Formel IIa 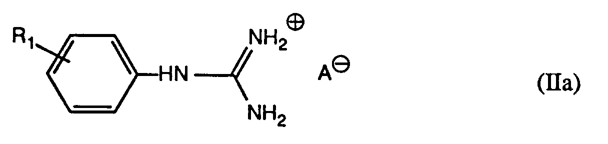 oder eine Guanidinverbindung der Formel IIb 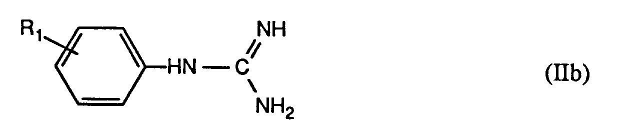 mit einem Keton der Formel III 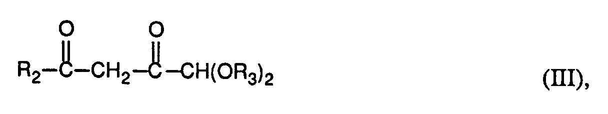 worin R₃ ein Alkyl bedeutet, in einem protischen Lösungsmittel oder ohne Lösungsmittel bei Temperaturen von 40° bis 160°C zu einer Pyrimidinverbindung der Formel IV 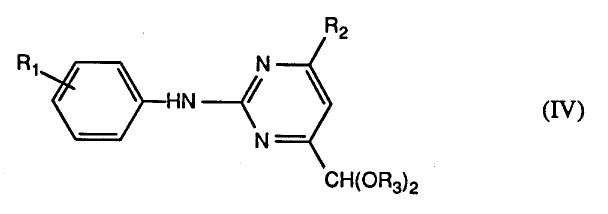 umsetzt und
1.2 das erhaltene Acetal der Formel IV in Gegenwart einer Säure in Wasser oder wässrigen Gemischen von inerten Lösungsmitteln bei Temperaturen von 20° bis 100°C zum Pyrimidinylaldehyd der Formel V 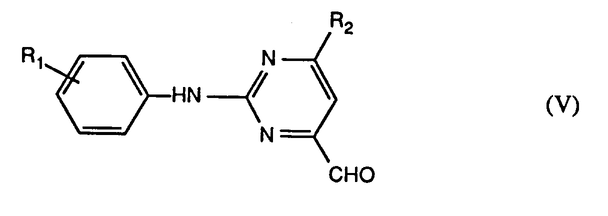 hydrolysiert und
1.3 anschliessend den Aldehyd der Formel V mit einem Hydroxylaminsalz und einer Base, die das Hydroxylamin freisetzt, in einem protischen Lösungsmittel bei Temperaturen von 10° bis 90°C, in das Oxim der Formel VI 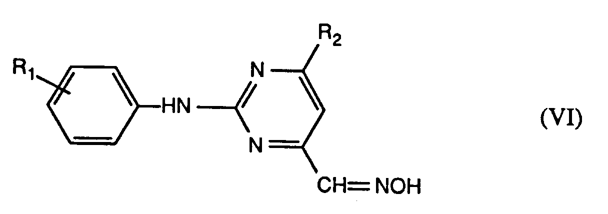 überführt und
1.3.1 das Oxim der Formel VI einer Wasserabspaltung unterwirft; wobei das Oxim intermediär zu einer Verbindung der Formel VII 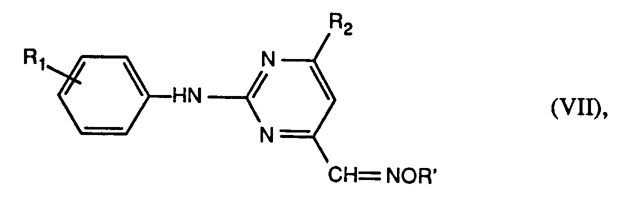 worin R' einen Acylrest COR'', einen Carbamoylrest CONHR'' oder einen Oxycarbonylrest COOR'' darstellt, in welchem R'' einen Alkylrest bedeutet, umgewandelt wird und anschliessend die Verbindung der Formel VII durch Abspaltung des Acylrestes HOCOR'' in inerten Lösungsmitteln bei Temperaturen von 60° bis 120°C, zersetzt; oder
1.4 aus dem Pyrimidinaldehyd der Formel V durch Umsetzung in tertiären Basen intermediär das Oxim der Formel VI herstellt und daraus durch Behandlung mit einem Säurehalogenid oder Säureanhydrid bei Temperaturen von 40° bis 120°C in situ direkt die Verbindung der Formel I erhalt; oder
2. eine Verbindung der Formel VIII 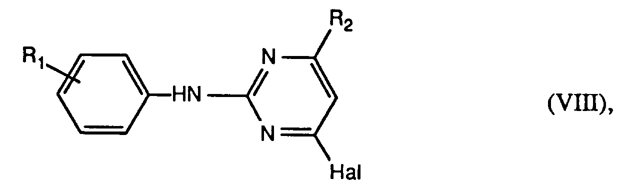 mit einem Cyanid der Formel IX
Me-CN (IX),
worin Hal Fluor, Chlor, Brom oder Jod bedeutet, und Me ein Alkali-, Erdalkali- oder Schwermetallkation darstellt, in einem aprotischen Lösungsmittel bei Temperaturen von 60° bis 160°C umsetzt; oder
3.1 Harnstoff der Formel X 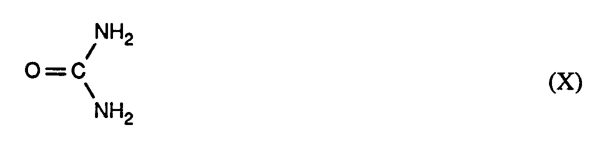 mit einem Diketon der Formel III 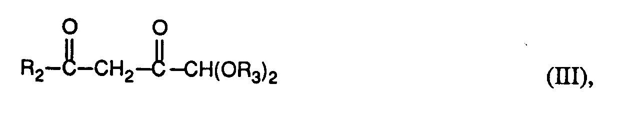 worin R₃ ein Alkyl bedeutet, in Gegenwart einer Säure in einem inerten Lösungsmittel bei Temperaturen von 20° bis 140°C zu einer Pyrimidinverbindung der Formel XI 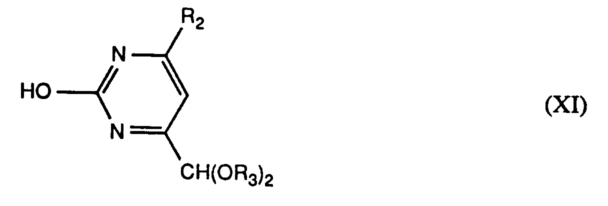 cyclisiert und
3.2 die OH-Gruppe der Verbindung der Formel XI mit überschüssigem PO(Hal)₃ in Gegenwart oder Abwesenheit eines inerten Lösungsmittels bei Temperaturen von 50° bis 110°C gegen Halogen austauscht und eine Verbindung der Formel XII 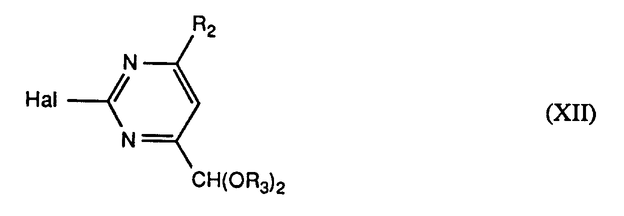 erhält, wobei Hal Halogen bedeutet und weiter
3.3 eine Verbindung der Formel XII mit einer Anilinverbindung der Formel XIII 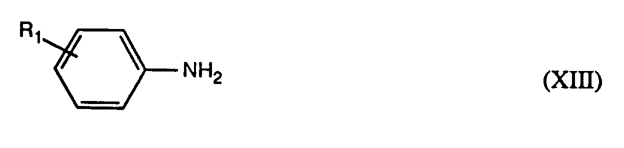 entweder
a) in Gegenwart eines Protonenakzeptors mit oder ohne inertes Lösungsmittel oder
b) in Gegenwart einer Säure in einem inerten Lösungsmittels bei jeweils Temperaturen von 60° bis 120°C zu einer Verbindung der Formel IV 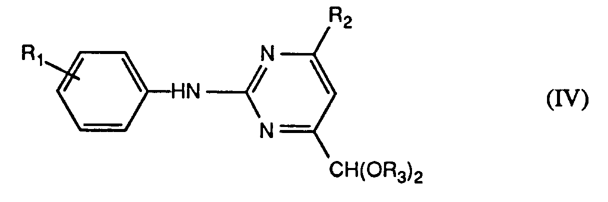 umsetzt und das erhaltene Acetal wie unter 1.2, 1.3 und 1.3.1 beschrieben in eine Verbindung der Formel I überführt, wobei in den vorgängig beschriebenen Verfahren die Reste R₁ und R₂ die unter Formel I angegebenen Bedeutungen besitzen.

7. Mittel zum Schutz von Pflanzen gegen den Befall durch Mikroorganismen, dadurch gekennzeichnet, dass es als aktive Komponente mindestens eine Verbindung gemäss Anspruch 1 enthält.

8. Mittel gemäss Anspruch 7, dadurch gekennzeichnet, dass dieses zusätzlich mindestens noch einen Trägerstoff enthält.

9. Mittel gemäss Anspruch 7, dadurch gekennzeichnet, dass es als aktive Komponente mindestens eine Verbindung gemäss den Ansprüchen 2 bis 4 enthält.

10. Mittel gemäss Anspruch 7, dadurch gekennzeichnet, dass es als aktive Komponente eine Verbindung der Formel I gemäss Anspruch 5 enthält.

11. Verfahren zur Herstellung eines agrochemischen Mittels von Anspruch 7, dadurch gekennzeichnet, dass man mindestens eine gemäss Anspruch 1 definierte Verbindung mit geeigneten festen oder flüssigen Träger- und Hilfsstoffen innig vermischt.

12. Verwendung von Verbindungen gemäss Anspruch 1 zum Schutz von Pflanzen gegen den Befall durch phytopathogene Mikroorganismen.

13. Verwendung von Verbindungen gemäss einem der Ansprüche 2 bis 5 zum Schutz von Pflanzen gegen den Befall durch phytopathogene Mikroorganismen.

14. Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

15. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 5 auf die Pflanze oder deren Standort appliziert.

16. Verfahren gemäss den Ansprüchen 14 und 15, dadurch gekennzeichnet, dass es sich bei den phytopathogenen Mikroorganismen um Pilzorganismen handelt.

## Patentansprüche (Patentansprüche für folgende(n) Vertragsstaat(en): ES)

1. Verfahren zur Herstellung der Verbindungen der Formel I 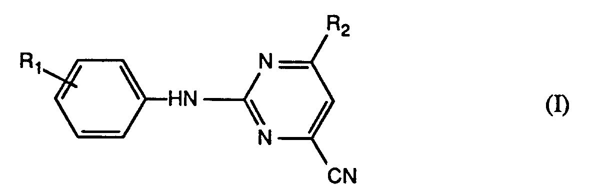 in welcher bedeuten:
R₁ Wasserstoff, 3-Fluor oder 4-Fluor; und
R₂ C₁-C₄-Alkyl, durch Halogen oder Hydroxy substituiertes C₁-C₂-Alkyl, C₃-C₆-Cycloalkyl oder durch Methyl und/oder Halogen ein- bis dreifach gleich oder verschieden substituiertes C₃-C₆-Cycloalkyl; unter Einschluss ihrer Säureadditionssalze und Metallsalzkomplexe, dadurch gekennzeichnet, dass man
1.1 ein Guanidinsalz der Formel IIa 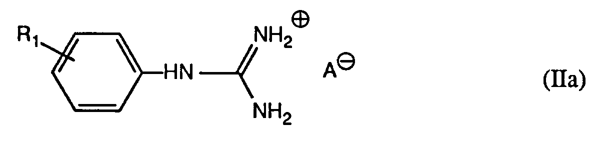 oder eine Guanidinverbindung der Formel IIb 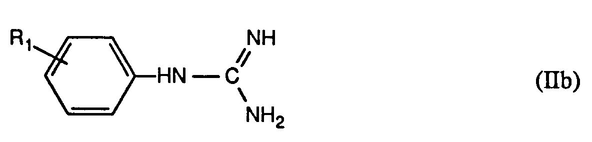 mit einem Keton der Formel III 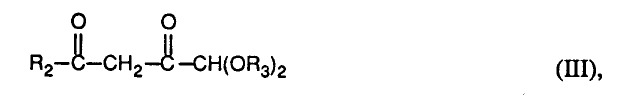 worin R₃ ein Alkyl bedeutet, in einem protischen Lösungsmittel oder ohne Lösungsmittel bei Temperaturen von 40° bis 160°C zu einer Pyrimidinverbindung der Formel IV 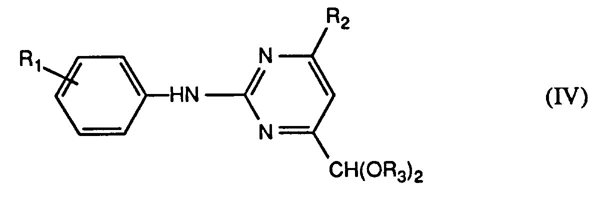 umsetzt und
1.2 das erhaltene Acetal der Formel IV in Gegenwart einer Säure in Wasser oder wässrigen Gemischen von inerten Lösungsmitteln bei Temperaturen von 20° bis 100°C zum Pyrimidinylaldehyd der Formel V 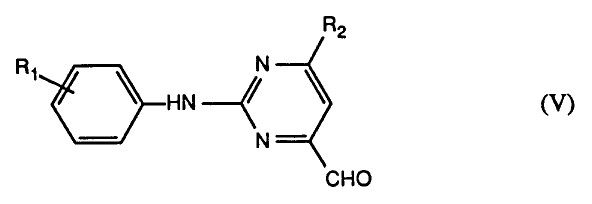 hydrolysiert und
1.3 anschliessend den Aldehyd der Formel V mit einem Hydroxylaminsalz und einer Base, die das Hydroxylamin freisetzt, in einem protischen Lösungsmittel bei Temperaturen von 10° bis 90°C, in das Oxim der Formel VI 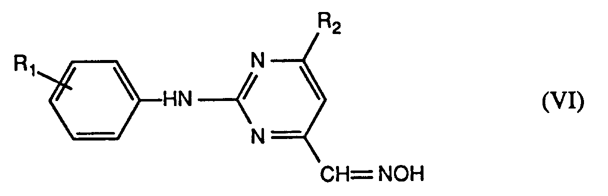 überführt und
1.3.1 das Oxim der Formel VI einer Wasserabspaltung unterwirft; wobei das Oxim intermediär zu einer Verbindung der Formel VII 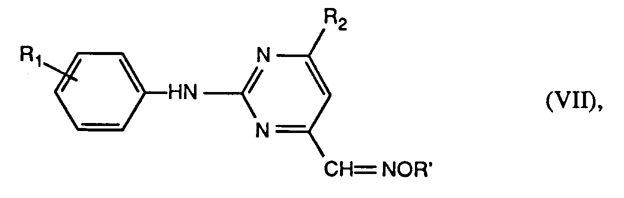 worin R' einen Acylrest COR'', einen Carbamoylrest CONHR'' oder einen Oxycarbonylrest COOR'' darstellt, in welchem R'' einen Alkylrest bedeutet, umgewandelt wird und anschliessend die Verbindung der Formel VII durch Abspaltung des Acylrestes HOCOR'' in inerten Lösungsmitteln bei Temperaturen von 60° bis 120°C, zersetzt; oder
1.4 aus dem Pyrimidinaldehyd der Formel V durch Umsetzung in tertiären Basen intermediär das Oxim der Formel VI herstellt und daraus durch Behandlung mit einem Säurehalogenid oder Säureanhydrid bei Temperaturen von 40° bis 120°C in situ direkt die Verbindung der Formel I erhält; oder
eine Verbindung der Formel VIII 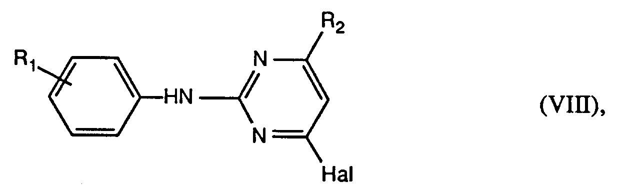 mit einem Cyanid der Formel IX
Me-CN (IX),
worin Hal Fluor, Chlor, Brom oder Jod bedeutet, und Me ein Alkali-, Erdalkali- oder Schwermetallkation darstellt, in einem aprotischen Lösungsmittel bei Temperaturen von 60° bis 160°C umsetzt; oder
Harnstoff der Formel X 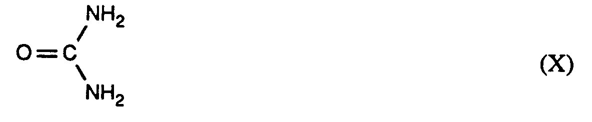 mit einem Diketon der Formel III 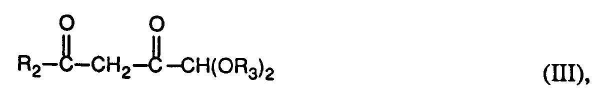 worin R₃ ein Alkyl bedeutet, in Gegenwart einer Säure in einem inerten Lösungsmittel bei Temperaturen von 20° bis 140°C zu einer Pyrimidinverbindung der Formel XI 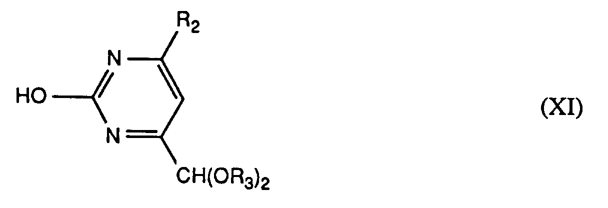 cyclisiert und
die OH-Gruppe der Verbindung der Formel XI mit überschüssigem PO(Hal)₃ in Gegenwart oder Abwesenheit eines inerten Lösungsmittels bei Temperaturen von 50° bis 110°C gegen Halogen austauscht und eine Verbindung der Formel XII 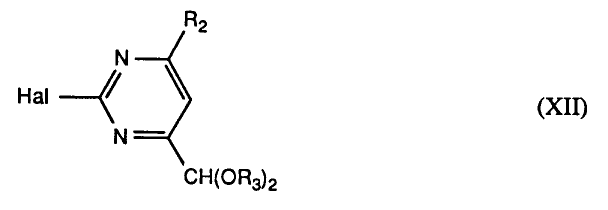 erhält, wobei Hal Halogen bedeutet und weiter
eine Verbindung der Formel XII mit einer Anilinverbindung der Formel XIII 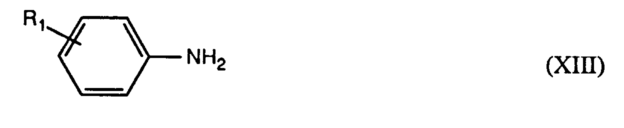 entweder
a) in Gegenwart eines Protonenakzeptors mit oder ohne inertes Lösungsmittel oder
b) in Gegenwart einer Säure in einem inerten Lösungsmittels bei jeweils Temperaturen von 60° bis 120°C zu einer Verbindung der Formel IV 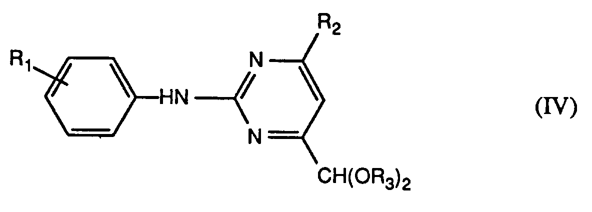 umsetzt und das erhaltene Acetal wie unter 1.2, 1.3 und 1.3.1 beschrieben in eine Verbindung der Formel I überführt, wobei in den vorgängig beschriebenen Verfahren die Reste R₁ und R₂ die unter Formel I angegebenen Bedeutungen besitzen.

2. Verfahren gemäss Anspruch 1, dadurch gekennzeichnet, dass man Verbindungen der Formel I, in welcher bedeuten:
R₁ Wasserstoff, 3-Fluor oder 4-Fluor; und
R₂ C₁-C₃-Alkyl, Cyclopropyl oder mit Methyl oder Chlor substituiertes Cyclopropyl;
herstelt.

3. Verfahren gemäss Anspruch 1, dadurch gekennzeichnet, dass man Verbindungen der Formel I, in welcher bedeuten:
R₁ Wasserstoff; und
R₂ Methyl, Ethyl, Cyclopropyl oder 2-Methylcyclopropyl
herstellt.

4. Verfahren gemäss Anspruch 1, dadurch gekennzeichnet, dass man Verbindungen der Formel I, in welcher bedeuten:
R₁ Wasserstoff, 3-Fluor oder 4-Fluor; und
R₂ Methyl, Ethyl oder Cyclopropyl;
herstellt.

5. Verfahren gemäss Anspruch 1, dadurch gekennzeichnet, dass man eine Verbindung ausgewählt aus der Gruppe
2-Phenylamino-4-cyano-6-methyl-pyrimidin (Verb. Nr. 1.1),
2-Phenylamino-4-cyano-6-ethyl-pyrimidin (Verb. Nr. 1.3),
2-Phenylamino-4-cyano-6-cyclopropyl-pyrimidin (Verb. Nr. 1.11) und
2-(3-Fluorphenylamino)-4-cyano-6-methyl-pyrimidin (Verb. Nr. 1.16)
herstellt.

6. Mittel zum Schutz von Pflanzen gegen den Befall durch Mikroorganismen, dadurch gekennzeichnet, dass es als aktive Komponente mindestens eine Verbindung der Formel 1 von Anspruch 1 enthält.

7. Mittel gemäss Anspruch 6, dadurch gekennzeichnet, dass dieses zusätzlich mindestens noch einen Trägerstoff enthält.

8. Mittel gemäss Anspruch 6, dadurch gekennzeichnet, dass es als aktive Komponente mindestens eine Verbindung der Formel 1 der Ansprüche 2 bis 4 enthält.

9. Mittel gemäss Anspruch 6, dadurch gekennzeichnet, dass es als aktive Komponente eine Verbindung der Formel I von Anspruch 5 enthält.

## Claims (Claims for the following Contracting State(s): AT, BE, CH, DE, DK, FR, GB, GR, IT, LI, LU, NL, SE)

1. A compound of formula I 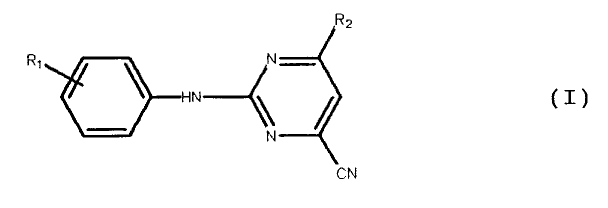 wherein:
R₁ is hydrogen, 3-fluorine or 4-fluorine, and
R₂ is C₁-C₄ alkyl, halo- or hydroxy-substituted C₁-C₂ alkyl, C₃-C₆ cycloalkyl or C₃-C₆ cycloalkyl mono- to tri-substituted by identical or different substituents selected from methyl and halogen;
or an acid addition salt or metal salt complex thereof.

2. A compound of formula I according to claim 1 wherein:
R₁ is hydrogen, 3-fluorine or 4-fluorine, and
R₂ is C₁-C₃ alkyl, cyclopropyl or cyclopropyl substituted by methyl or by chlorine.

3. A compound of formula I according to claim 1 wherein:
R₁ is hydrogen and
R₂ is methyl, ethyl, cyclopropyl or 2-methylcyclopropyl.

4. A compound of formula I according to claim 1 wherein:
R₁ is hydrogen, 3-fluorine or 4-fluorine, and
R₂ is methyl, ethyl or cyclopropyl.

5. A compound of formula I according to claim 4 selected from the group:
2-phenylamino-4-cyano-6-methylpyrimidine (comp. no. 1.1),
2-phenylamino-4-cyano-6-ethylpyrimidine (comp. no. 1.3),
2-phenylamino-4-cyano-6-cyclopropylpyrimidine (comp. no. 1.11) and
2-(3-fluorophenylamino)-4-cyano-6-methylpyrimidine (comp. no. 1.16).

6. A process for the preparation of a compound of formula I, which process comprises
1.1 reacting a guanidine salt of formula IIa 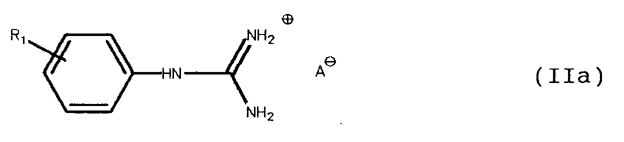 or a guanidine compound of formula IIb 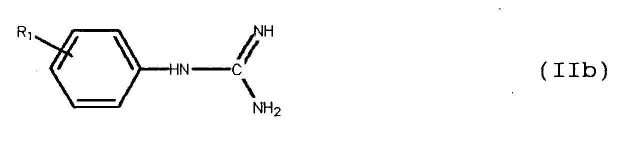 with a ketone of formula III 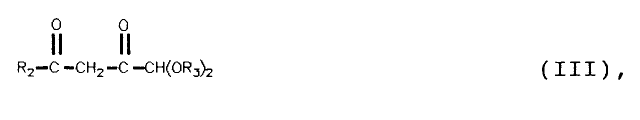 wherein R₃ is an alkyl radical, in a protic solvent or without a solvent, at temperatures of from 40° to 160°C, to form a pyrimidine compound of formula IV 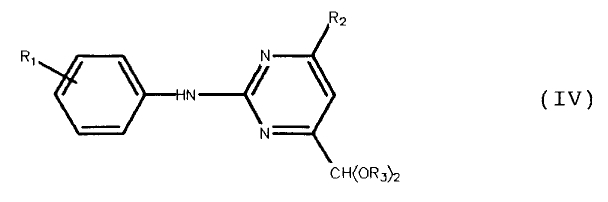 and
1.2 hydrolysing the resulting acetal of formula IV in the presence of an acid, in water or aqueous mixtures of inert solvents, at temperatures of from 20° to 100°C, to form the pyrimidinylaldehyde of formula V 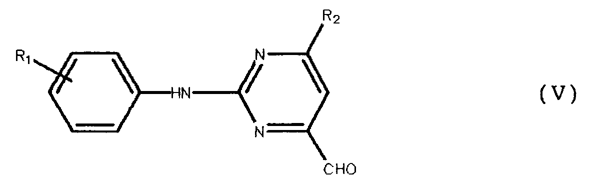 and
1.3 then converting the aldehyde of formula V with a hydroxylamine salt and a base that frees the hydroxylamine, in a protic solvent, at temperatures of from 10° to 90°C, into the oxime of formula VI 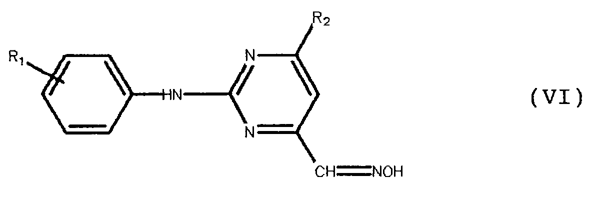 and
1.3.1 subjecting the oxime of formula VI to a reaction in which the elements of water are removed; the oxime being converted in an intermediate step into a compound of formula VII 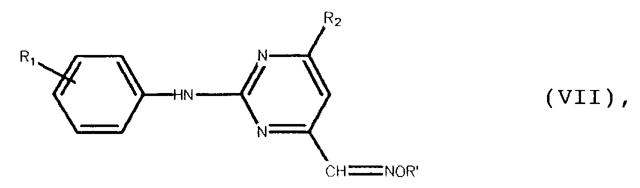 wherein R' is an acyl radical COR'', a carbamoyl radical CONHR'' or an oxycarbonyl radical COOR'', in which R'' is an alkyl radical, and the compound of formula VII then being decomposed by removal of the acyl radical HOCOR'' in inert solvents at temperatures of from 60° to 120°C; or
1.4 from the pyrimidinealdehyde of formula V, preparing the oxime of formula VI in an intermediate step by reaction in tertiary bases, and forming the compound of formula I directly therefrom in situ by treatment with an acid halide or acid anhydride at temperatures of from 40° to 120°C; or
2. reacting a compound of formula VIII 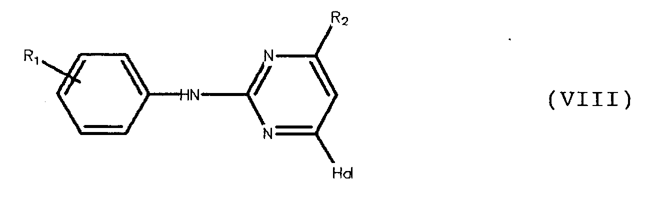 with a cyanide of formula IX
Me-CN (IX),
wherein Hal is fluorine, chlorine, bromine or iodine and Me is an alkali metal cation, alkaline earth metal cation or heavy metal cation, in an aprotic solvent at temperatures of from 60° to 160°C; or
3.1 cyclising urea of formula X 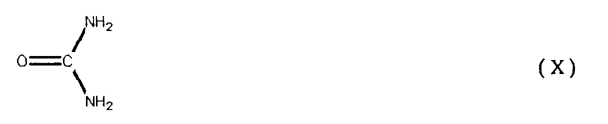 with a diketone of formula III 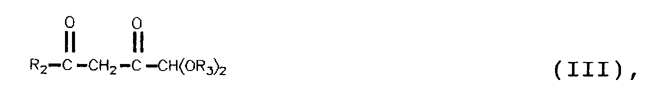 wherein R₃ is an alkyl radical, in the presence of an acid in an inert solvent at temperatures of from 20° to 140°C, to form a pyrimidine compound of formula XI 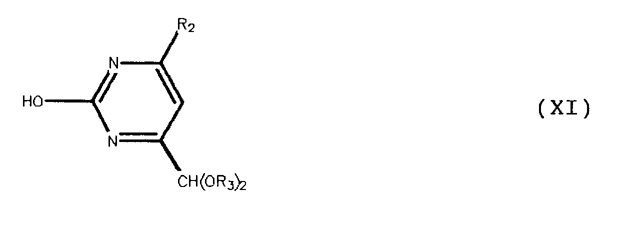 and
3.2 replacing the OH group in the compound of formula XI by halogen using excess PO(Hal)₃ in the presence or absence of an inert solvent at temperatures of from 50° to 110°C to form a compound of formula XII 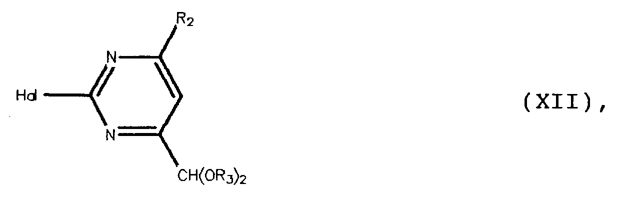 wherein Hal is halogen, and then
3.3 reacting a compound of formula XII with an aniline compound of formula XIII 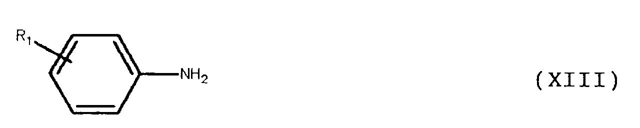 either
a) in the presence of a proton acceptor, with or without an inert solvent, or
b) in the presence of an acid in an inert solvent, in each case at temperatures of from 60° to 120°C, to form a compound of formula IV 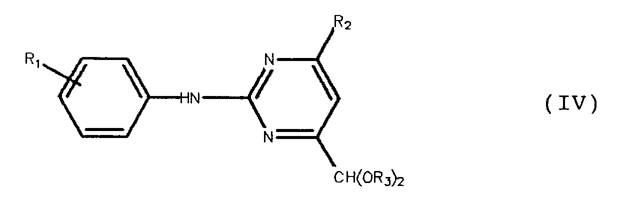 and converting the resulting acetal into a compound of formula I in the manner described under 1.2, 1.3 and 1.3.1, the radicals R₁ and R₂ in the above-described processes being as defined under formula I.

7. A composition for protecting plants from attack by microorganisms, which comprises as active ingredient at least one compound according to claim 1.

8. A composition according to claim 7, which comprises in addition at least one carrier.

9. A composition according to claim 7, which comprises as active ingredient at least one compound according to claims 2 to 4.

10. A composition according to claim 7, which comprises as active ingredient a compound of formula I according to claim 5.

11. A process for the preparation of an agrochemical composition according to claim 7, which process comprises homogeneously mixing at least one compound defined according to claim 1 with suitable solid or liquid carriers and adjuvants.

12. The use of a compound according to claim 1 for protecting plants against attack by phytopathogenic microorganisms.

13. The use of a compound according to any one of claims 2 to 5 for protecting plants against attack by phytopathogenic microorganisms.

14. A method of protecting plants against attack by phytopathogenic microorganisms, which comprises applying a compound according to claim 1 as active ingredient to the plant or to the locus thereof.

15. A method of protecting plants against attack by phytopathogenic microorganisms, which comprises applying a compound according to any one of claims 2 to 5 as active ingredient to the plant or to the locus thereof.

16. A method according to claims 14 and 15, wherein the phytopathogenic microorganisms are fungus organisms.

## Claims (Claims for the following Contracting State(s): ES)

1. A process for the preparation of a compound of formula I 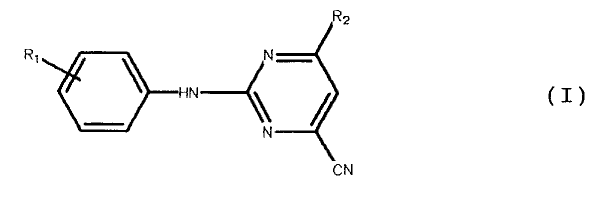 wherein:
R₁ is hydrogen, 3-fluorine or 4-fluorine, and
R₂ is C₁-C₄ alkyl, halo- or hydroxy-substituted C₁-C₂ alkyl, C₃-C₆ cycloalkyl or C₃-C₆cycloalkyl mono- to tri-substituted by identical or different substituents selected from methyl and halogen;
or of an acid addition salt or metal salt complex thereof, which process comprises
1.1 reacting a guanidine salt of formula IIa 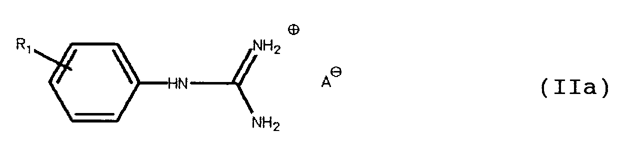 or a guanidine compound of formula IIb 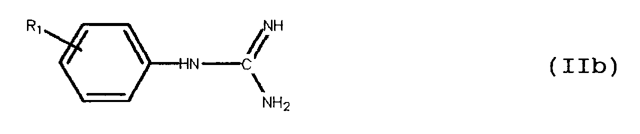 with a ketone of formula III 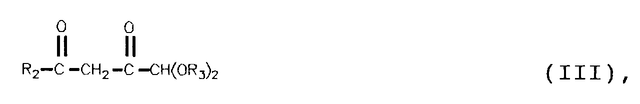 wherein R₃ is an alkyl radical, in a protic solvent or without a solvent, at temperatures of from 40° to 160°C, to form a pyrimidine compound of formula IV 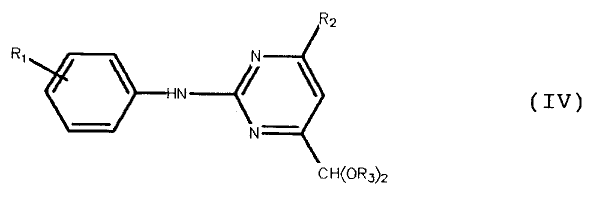 and
1.2 hydrolysing the resulting acetal of formula IV in the presence of an acid, in water or aqueous mixtures of inert solvents, at temperatures of from 20° to 100°C, to form the pyrimidinylaldehyde of formula V 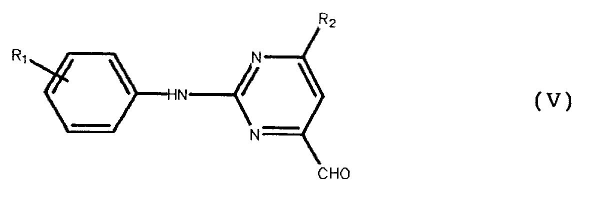 and
1.3 then converting the aldehyde of formula V with a hydroxylamine salt and a base that frees the hydroxylamine, in a protic solvent, at temperatures of from 10° to 90°C, into the oxime of formula VI 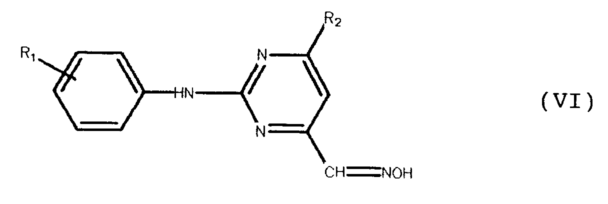 and
1.3.1 subjecting the oxime of formula VI to a reaction in which the elements of water are removed; the oxime being converted in an intermediate step into a compound of formula VII 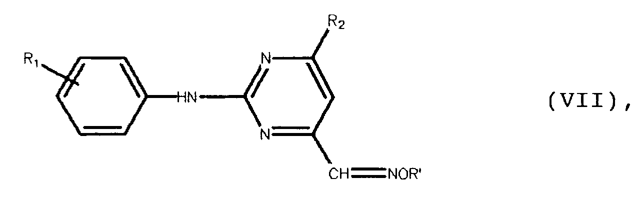 wherein R' is an acyl radical COR'', a carbamoyl radical CONHR'' or an oxycarbonyl radical COOR'', in which R'' is an alkyl radical, and the compound of formula VII then being decomposed by removal of the acyl radical HOCOR'' in inert solvents at temperatures of from 60° to 120°C; or
1.4 from the pyrimidinealdehyde of formula V, preparing the oxime of formula VI in an intermediate step by reaction in tertiary bases, and forming the compound of formula I directly therefrom in situ by treatment with an acid halide or acid anhydride at temperatures of from 40° to 120°C; or
reacting a compound of formula VIII 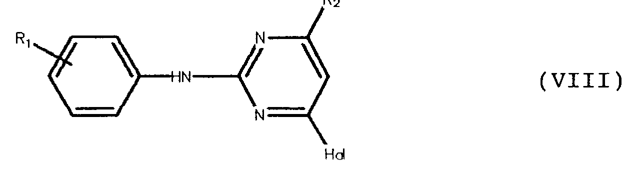 with a cyanide of formula IX
Me-CN (IX),
wherein Hal is fluorine, chlorine, bromine or iodine and Me is an alkali metal cation, alkaline earth metal cation or heavy metal cation, in an aprotic solvent at temperatures of from 60° to 160°C; or
cyclising urea of formula X 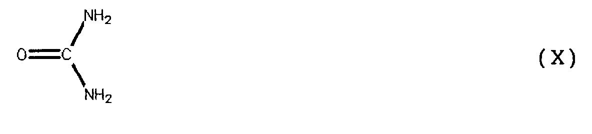 with a diketone of formula III 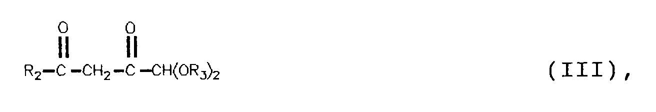 wherein R₃ is an alkyl radical, in the presence of an acid in an inert solvent at temperatures of from 20° to 140°C, to form a pyrimidine compound of formula XI 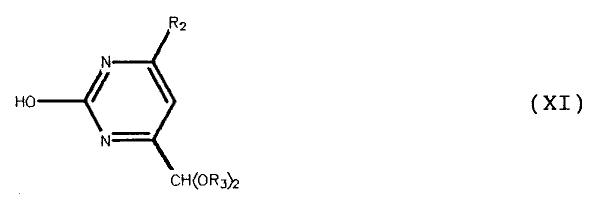 and
replacing the OH group in the compound of formula XI by halogen using excess PO(Hal)₃ in the presence or absence of an inert solvent at temperatures of from 50° to 110°C to form a compound of formula XII 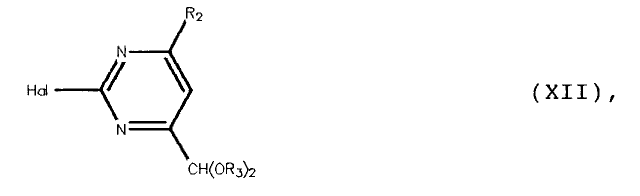 wherein Hal is halogen, and then
reacting a compound of formula XII with an aniline compound of formula XIII 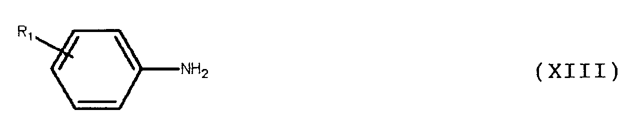 either
a) in the presence of a proton acceptor, with or without an inert solvent, or
b) in the presence of an acid in an inert solvent, in each case at temperatures of from 60° to 120°C, to form a compound of formula IV 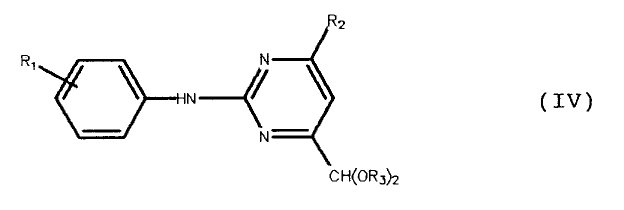 and converting the resulting acetal into a compound of formula I in the manner described under 1.2, 1.3 and 1.3.1, the radicals R₁ and R₂ in the above-described processes being as defined under formula I.

2. A process according to claim 1 wherein there is prepared a compound of formula I wherein:
R₁ is hydrogen, 3-fluorine or 4-fluorine, and
R₂ is C₁-C₃alkyl, cyclopropyl or cyclopropyl substituted by methyl or by chlorine.

3. A process according to claim 1 wherein there is prepared a compound of formula I wherein:
R₁ is hydrogen and
R₂ is methyl, ethyl, cyclopropyl or 2-methylcyclopropyl.

4. A process according to claim 1 wherein there is prepared a compound of formula I wherein:
R₁ is hydrogen, 3-fluorine or 4-fluorine, and
R₂ is methyl, ethyl or cyclopropyl.

5. A process according to claim 1 wherein there is prepared a compound selected from the group:
2-phenylamino-4-cyano-6-methylpyrimidine (comp. no. 1.1),
2-phenylamino-4-cyano-6-ethylpyrimidine (comp. no. 1.3),
2-phenylamino-4-cyano-6-cyclopropylpyrimidine (comp. no. 1.11) and
2-(3-fluorophenylamino)-4-cyano-6-methylpyrimidine (comp. no. 1.16).

6. A composition for protecting plants from attack by microorganisms, which comprises as active ingredient at least one compound of formula I according to claim 1.

7. A composition according to claim 6, which comprises in addition at least one carrier.

8. A composition according to claim 6, which comprises as active ingredient at least one compound of formula I according to claims 2 to 4.

9. A composition according to claim 6, which comprises as active ingredient a compound of formula I according to claim 5.

## Revendications (Revendications pour l'(les) Etat(s) contractant(s) suivant(s): AT, BE, CH, DE, DK, FR, GB, GR, IT, LI, LU, NL, SE)

1. Composés de formule I 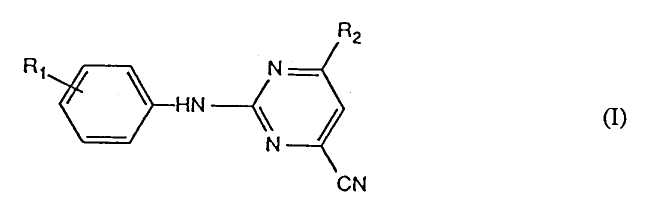 dans laquelle :
R₁ représente un hydrogène, un 3-fluoro ou 4-fluoro : et
R₂ est un alkyle C₁₋₄, un alkyle C₁₋₂ substitué par un halogène ou un hydroxy, un cycloalkyle C₃₋₆ ou un cycloalkyle C₃₋₆ substitué de une à trois fois par des substituants identiques ou différents qui sont le méthylène et/ou un halogène ; en tenant compte de leurs sels d'addition d'acides et leurs complexes métalliques.

2. Composés de formule I selon la revendication 1, où
R₁ représente un hydrogène, un 3-fluoro ou un 4-fluoro ; et
R₂ est un alkyle C₁₋₃, cyclopropyle ou un cyclopropyle substitué par un méthyle ou un chlore.

3. Composés de formule I selon la revendication 1, où:
R₁ est un hydrogène ; et
R₂ est un méthyle, éthyle, cyclopropyle ou 2-méthylcyclopropyle.

4. Composés de formule I selon la revendication 1, où:
R₁ est un hydrogène, un 3-fluoro ou un 4-fluoro, et
R₂ est un méthyle, éthyle ou cyclopropyle.

5. Composé de formule I selon la revendication 4 choisi dans le groupe :
2-phénylamino-4-cyano-6-méthyl-pyrimidine (composé n° 1.1)
2-phénylamino-4-cyano-6-éthyl-pyrimidine (composé n° 1.3)
2-phénylamino-4-cyano-6-cyclopropyl-pyrimidine (composé n° 1.11) et
2-(3-fluorophénylamino)-4-cyano-6-méthyl-pyrimidine (composé n° 1.16).

6. Procédé de préparation des composés de formule I, caractérisé en ce que
1.1. on fait réagir un sel de guanidine de formule IIa 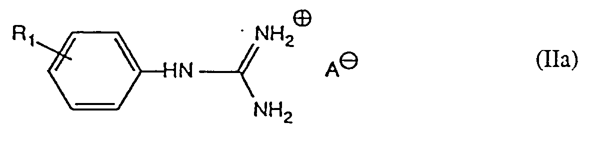 ou un composé de guanidine de formule IIb 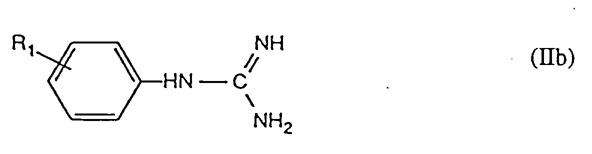 avec une cétone de formule III 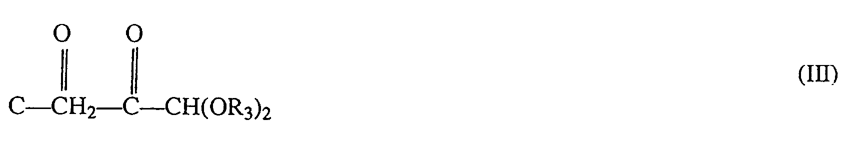 dans laquelle R₃ est un alkyle, dans un solvant protique ou sans solvant à des températures de 40° à 160°C pour donner un composé pyrimidine de formule IV 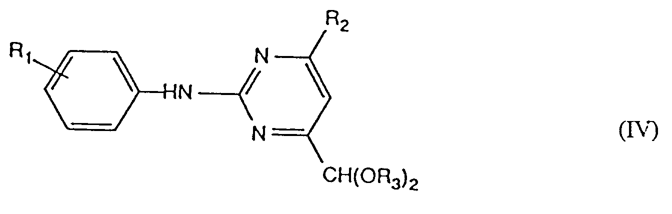 et
1.2. on hydrolyse l'acétal obtenu de formule IV en présence d'un acide dans l'eau ou des mélanges aqueux de solvants inertes à des températures de 20° à 100°C pour donner le pyrimidinylaldéhyde de formule V 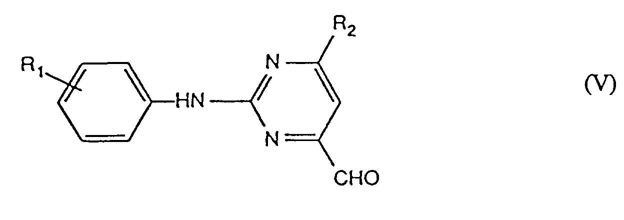 et
1.3. ensuite, on transforme l'aldéhyde de formule V avec un sel d'hydroxylamine et une base qui libère l'hydroxylamine, dans un solvant protique, à des températures de 10° à 90°C, en l'oxime de formule VI 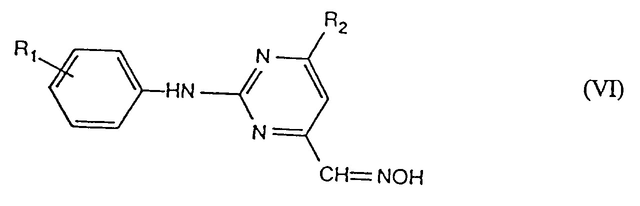 et
1.3.1. on soumet l'oxime de formule VI à une élimination d'eau ; où on forme l'oxime par exemple en intermédiaire en un composé de formule VII 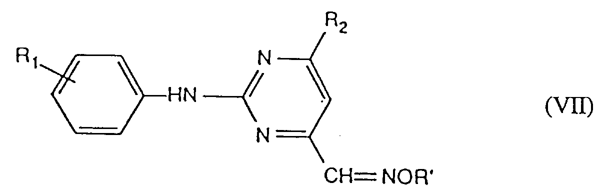 où R' est un reste acyle COR'', un reste carbamoyle CONHR'', ou un reste oxycarbonyle COOR'', dans lequel R'' est un reste alkyle et ensuite on décompose le composé de formule VII par élimination du reste acyle HOCOR'' dans un solvant inerte à des températures de 60° à 120°C ; ou
1.4. à partir du pyrimidinaldéhyde de formule V par réaction dans des bases tertiaires, on prépare de façon intermédiaire l'oxime de formule VI et on obtient par traitement avec un halogénure d'acide ou un anhydride d'acide à des températures de 40° à 120°C in situ directement le composé de formule I ; ou
2. Un composé de formule VIII 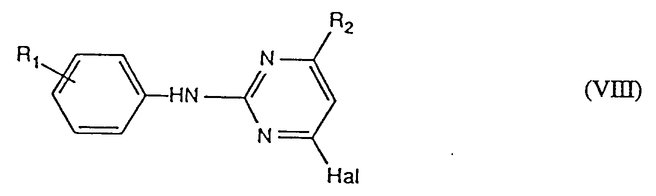 est mis à réagir avec un cyanure de formule IX
Me-CN (IX)
où Hal est le fluor, chlore, brome ou iode et Me est un cation alcalin, alcalinoterreux ou de métal lourd, dans un solvant aprotique à des températures de 60° à 160°C ; ou 3.1. on cyclise une urée de formule X 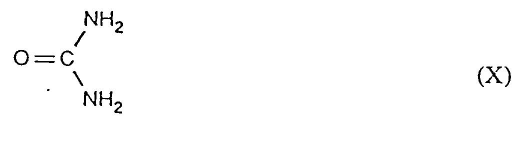 avec une dicétone de formule III 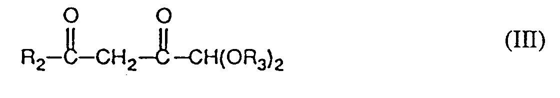 où R₃ est un alkyle, en présence d'un acide dans un solvant inerte à des températures de 20° à 140°C pour donner un composé pyrimidine de formule XI 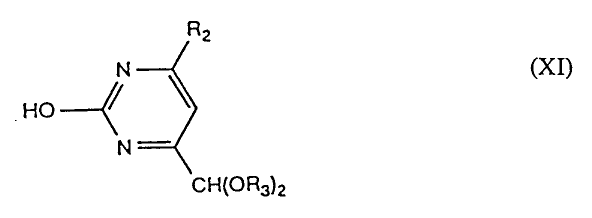 et
3.2. on remplace par un halogène le groupe OH du composé de formule XI avec un excès de PO(Hal)₃ en présence ou en l'absence d'un solvant inerte à des températures de 500 à 110°C et on obtient un composé de formule XII 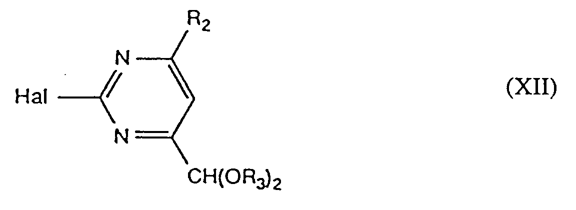 où Hal représente un halogène et ensuite 3.3. on fait réagir un composé de formule XII avec un composé d'aniline de formule XIII 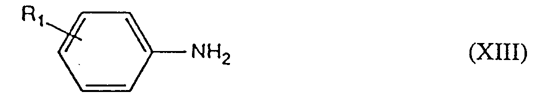 soit
a) en présence d'un accepteur de protons avec ou sans solvant inerte ou b) en présence d'un acide dans un solvant inerte aux températures de 60° à 120°C pour donner un composé de formule IV 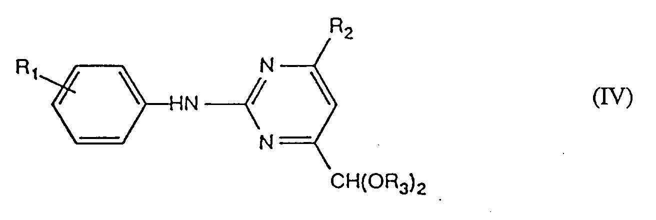 et on transforme l'acétal obtenu comme c'est décrit aux paragraphes 1.2, 1.3 et 1.3.1 en un composé de formule I, où dans le procédé déjà décrit, les restes R₁ et R₂ ont les significations données pour la formule I.

7. Moyen de protection des plantes contre l'attaque par les microorganismes, caractérisé en ce qu'il contient comme composants actifs au moins l'un des composés selon la revendication 1.

8. Moyen selon la revendication 7, caractérisé en ce que celui-ci contient en plus au moins un véhicule.

9. Moyen selon la revendication 7, caractérisé en ce qu'il contient comme composants actifs au moins un composé selon les revendications 2 à 4.

10. Moyen selon la revendication 7, caractérisé en ce qu'il contient comme composants actifs un composé de formule I selon la revendication 5.

11. Procédé de préparation d'un moyen agrochimique selon la revendication 7, caractérisé en ce qu'on mélange intimement au moins un composé défini selon la revendication 1 à des véhicules ou additifs solides ou liquides appropriés.

12. Utilisation de composés selon la revendication 1 pour protéger les plantes contre l'attaque par des microorganismes phytopathogènes.

13. Utilisation de composés selon l'une des revendications 2 à 5 pour protéger des plantes contre l'attaque par des microorganismes phytopathogènes.

14. Procédé de protection de plantes contre l'attaque par des microorganismes pathogènes, caractérisé en ce qu'on applique comme principe actif un composé selon la revendication 1 sur les plantes ou leur terrain.

15. Procédé de protection des plantes contre l'attaque par des microorganismes phytopathogènes, caractérisé en ce qu'on applique comme principe actif un composé selon l'une des revendications 2 à 5 sur les plantes ou leur terrain.

16. Procédé selon les revendications 14 et 15, caractérisé en ce qu'il s'agit d'organismes champignons pour les microorganismes phytopathogdnes.

## Revendications (Revendications pour l'(les) Etat(s) contractant(s) suivant(s): ES)

1. Procédé de préparation des composés de formule I 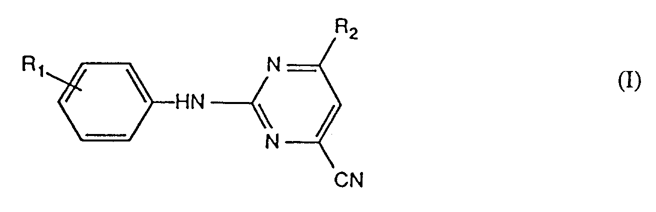 dans laquelle :
R₁ représente un hydrogène, un 3-fluoro ou un 4-fluoro ; et
R₂ est un alkyle C₁₋₄, un alkyle C₁₋₂ substitué par un halogène ou un hydroxy, un cycloalkyle en C₃₋₆ ou un cycloalkyle en C₃₋₆ substitué de une à trois fois par des substituants identiques ou différents qui sont le méthylène et/ou un halogène ; en tenant compte de leurs sels d'addition d'acides et leurs complexes métalliques,
caractérisé en ce que
1.1. on fait réagir un sel de guanidine de formule IIa 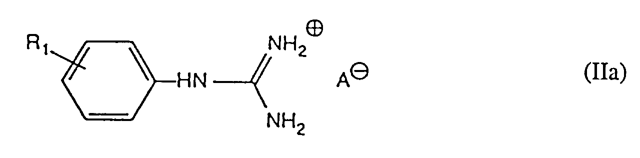 ou un composé de guanidine de formule IIb 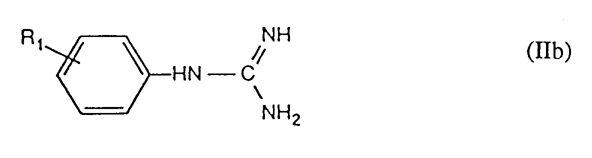 avec une cétone de formule III 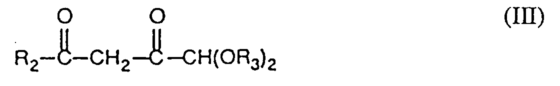 dans laquelle R₃ est un alkyle, dans un solvant protique ou sans solvant à des températures de 40° à 160°C pour donner un composé pyrimidine de formule IV 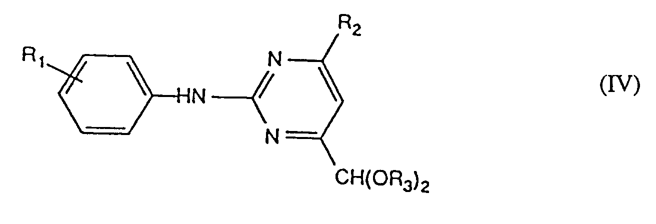 et
1.2. on hydrolyse l'acétal obtenu de formule IV en présence d'un acide dans l'eau ou des mélanges aqueux de solvants inertes à des températures de 20° à 100°C pour donner le pyrimidinylaldéhyde de formule V 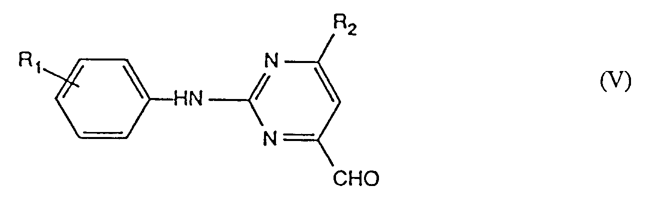 et
1.3. ensuite, on transforme l'aldéhyde de formule V avec un sel d'hydroxylamine et une base qui libère l'hydroxylamine, dans un solvant protique, à des températures de 10° à 90°C, en l'oxime de formule VI 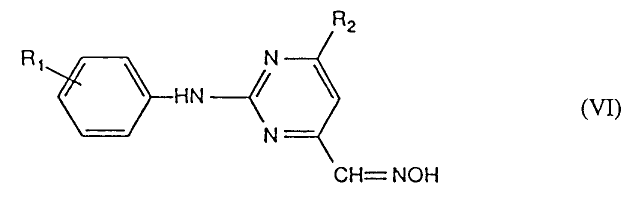 et
1.3.1 on soumet l'oxime de formule VI à une élimination d'eau ; où on forme l'oxime par exemple en intermédiaire en un composé de formule VII 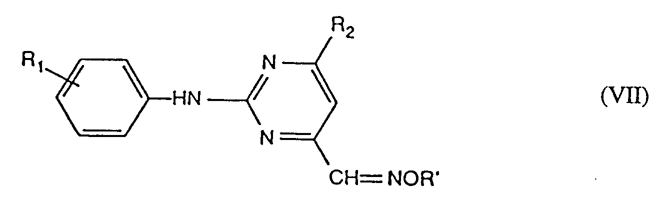 où R' est un reste acyle COR'', un reste carbamoyle CONHR'', ou un reste oxycarbonyle COOR'', dans lequel R'' est un reste alkyle et ensuite on décompose le composé de formule VII par élimination du reste acyle HOCOR'' dans un solvant inerte à des températures de 60° à 120°C ; ou
1.4. à partir du pyrimidinaldéhyde de formule V par réaction dans des bases tertiaires, on prépare de façon intermédiaire l'oxime de formule VI et on obtient par traitement avec un halogénure d'acide ou un anhydride d'acide à des températures de 40° à 120°C in situ directement le composé de formule I ; ou
un composé de formule VIII 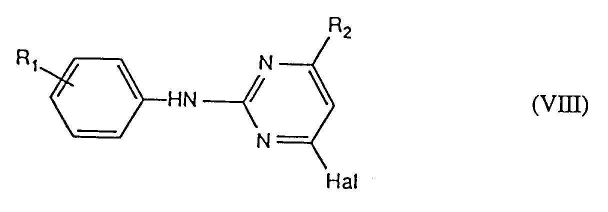 est mis à réagir avec un cyanure de formule IX
Me-CN (IX)
où Hal est le fluor, le chlore, le brome ou l'iode et Me est un cation alcalin, alcalino-terreux ou de métal lourd, dans un solvant aprotique à des températures de 60° à 160°C ; ou on cyclise une urée de formule X 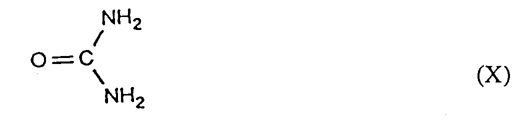 avec une dicétone de formule III 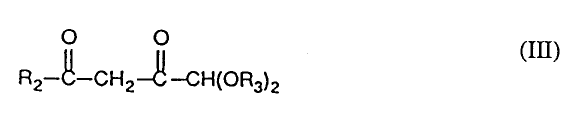 où R₃ est un alkyle, en présence d'un acide dans un solvant inerte à des températures de 20° à 140°C pour donner un composé pyrimidine de formule XI 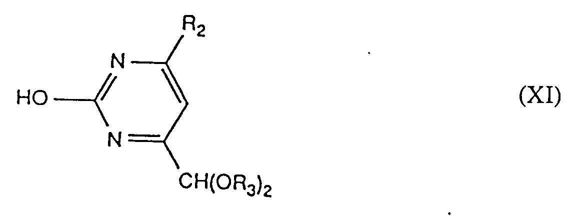 on remplace par un halogène le groupe OH du composé de formule XI avec un excès de PO(Hal)₃ en présence ou en l'absence d'un solvant inerte à des températures de 50° à 110°C et on obtient un composé de formule XII 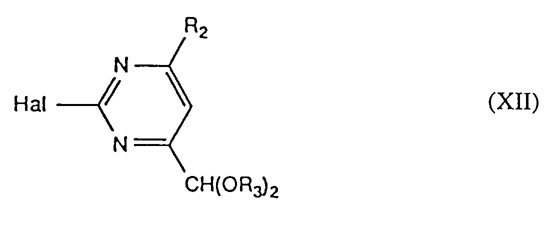 où Hal représente un halogène et ensuite on fait réagir un composé de formule XII avec un composé d'aniline de formule XIII 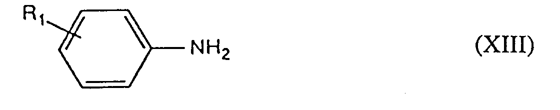 soit
a) en présence d'un accepteur de protons avec ou sans solvant inerte ou
b) en présence d'un acide dans un solvant inerte aux températures de 60° à 120°C pour donner un composé de formule IV 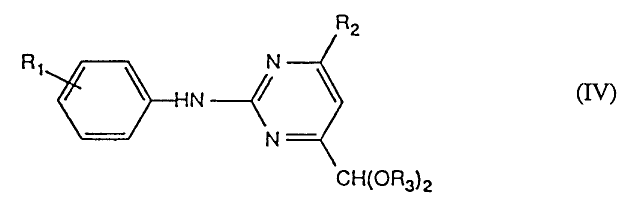 et on transforme l'acétal.obtenu comme c'est décrit aux paragraphes 1.2, 1.3 et 1.3.1 en un composé de formule I, où dans le procédé déjà décrit, les restes R₁ et R₂ ont les significations données pour la formule I.

2. Procédé selon la revendication 1, caractérisé en ce qu'on prépare des composés de formule I, où :
R₁ représente un hydrogène, un 3-fluoro ou un 4-fluoro ; et
R₂ est un alkyle C₁₋₃, cyclopropyle ou un cyclopropyle substitué par un méthyle ou un chlore.

3. Procédé selon la revendication 1, caractérisé en ce qu'on prépare des composés de formule I, où :
R₁ est un hydrogène ; et
R₂ est un méthyle, éthyle, cyclopropyle ou 2-méthylcyclopropyle.

4. Procédé selon la revendication 1, caractérisé en ce qu'on prépare des composés de formule I, où :
R₁ est un hydrogène, un 3-fluoro ou un 4-fluoro ; et R₂ est un méthyle, éthyle ou cyclopropyle.

5. Procédé selon la revendication 1, caractérisé en ce qu'on prépare un composé choisi dans le groupe :
2-phénylamino-4-cyano-6-méthyl-pyrimidine (composé n° 1.1),
2-phénylamino-4-cyano-6-éthyl-pyrimidine (composé n°1.3),
2-phénylamino-4-cyano-6-cyclopropyl-pyrimidine (composé n° 1.11) et
2-(3-fluorophénylamino)-4-cyano-6-méthyl-pyrimidine (composé n° 1.16).

6. Moyen de protection de plantes contre l'attaque par des microorganismes, caractérisé en ce qu'il contient comme composants actifs au moins un composé de formule I selon la revendication 1.

7. Moyen selon la revendication 6, caractérisé en ce que celui-ci contient au moins en plus un véhicule.

8. Moyen selon la revendication 6, caractérisé en ce qu'il contient comme composant actif au moins un composé de formule I selon les revendications 2 à 4.

9. Moyen selon la revendication 6, caractérisé en ce qu'il contient comme composant actif un composé de formule I selon la revendication 5.
